# EUROPÄISCHE PATENTSCHRIFT

(11) **EP 2 266 613 B1**
(45) Veröffentlichungstag und Bekanntmachung des Hinweises auf die Patenterteilung: **14.12.2016**
(21) Anmeldenummer: 10009751.8
(22) Anmeldetag: 23.09.2004
(51) Int. Cl.: C07K 14/705, A61K 39/395, C12N 5/10, C07K 14/47, C07K 16/30, C07K 16/32, A61K 48/00

(54) **IDENTIFIZIERUNG VON TUMORASSOZIIERTEN ZELLOBERFLÄCHEN-ANTIGENEN FÜR DIE DIAGNOSE UND THERAPIE**
IDENTIFICATION OF TUMOUR-ASSOCIATED CELL SURFACE ANTIGENS FOR DIAGNOSIS AND THERAPY
IDENTIFICATION D'ANTIGÈNES DE SURFACES CELLULAIRES ASSOCIÉS À DES TUMEURS POUR LE DIAGNOSTIC ET LA THÉRAPIE

(30) Priorität: 26.09.2003 DE 10344799
(43) Veröffentlichungstag der Anmeldung: 29.12.2010
(62) Teilanmeldung aus: 04786996.1
(73) Patentinhaber: BioNTech AG, 55131 Mainz (DE)
(72) Erfinder: Türeci, Özlem, 55116 Mainz (DE); Sahin, Ugur, 55116 Mainz (DE); Helftenbein, Gerd, 35329 Gemünden (Felda) (DE); Schlüter, Volker, 82061 Neuried (DE)
(74) Vertreter: Schnappauf, Georg

(56) Entgegenhaltungen:
- WO-A1-03/068816
- WO-A2-02/12343
- CA-A1- 2 418 130
- CA-A1- 2 478 413

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen interventionell verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. I präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionbanken von Tumoren systematisch auf Tumor-assoziierte Antigene zu screenen. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert (Tureci et al, Mol. Med. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Auch Serumreaktivitäten gegen CTA finden sich nicht in gesunden Kontrollen, sondern lediglich in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumorassoziiert exprimierter Antigene und der dafür kodierenden Nukleinsäuren verfolgt. Diese Strategie beruht auf der Auswertung humaner Protein- und Nukleinsäuredatenbanken im Hinblick auf potenzielle, auf der Zelloberfläche zugängliche, krebsspezifische Antigene. Die Definition der dafür notwendigen Filterkriterien zusammen mit einer Hochdurchsatz-Methodik zur Analyse möglichst aller Proteine bilden den zentralen Bestandteil der Erfindung. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken subtrahiert unter der Annahme, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Somit wird vorliegende eine Strategie zur Identifizierung von differentiell in Tumoren exprimierten Genen beschrieben. Diese kombiniert Datamining von öffentlichen Sequenzbanken ("*in silico*") mit darauffolgenden evaluierenden labor-experimentellen ("wet bench") Untersuchungen.

Eine kombinierte Strategie basierend auf unterschiedlichen bioinformatischen Skripten ermöglichte die Identifizierung des in Beispiel 3 beschriebenen Gens, das für auf der Zelloberfläche zugängliche, krebsspezifische Antigene kodiert. Die Identifizierung und Bereitstellung dieses Tumor-assoziierten Gens und der dadurch kodierten Genprodukte erfolgte erfindungsgemäß unabhängig von einer immunogenen Wirkung.

Der Schutzbereich dieser Erfindung wird durch die Patentansprüche näher definiert.

Die vorliegende Erfindung betrifft allgemein die Verwendung des erfindungsgemäß identifizierten Tumor-assoziierten Antigens oder von Teilen davon, von dafür kodierenden Nukleinsäuren oder von Nukleinsäuren, die gegen die kodierenden Nukleinsäuren gerichtet sind, oder von Antikörpern, die gegen das erfindungsgemäß identifizierte Tumor-assoziierte

Antigen oder Teile davon gerichtet sind, für die Therapie und Diagnose.

In einem Aspekt betrifft die Erfindung eine pharmazeutische Zusammensetzung, umfassend einen oder mehrere Bestandteile, die aus der Gruppe ausgewählt sind, bestehend aus:
(i) einem Tumor-assoziierten Antigen oder einem Teil davon, der mindestens 6 aufeinanderfolgende Aminosäuren des wie in (a) definierten Tumor-assoziierten Antigens umfasst,
(ii) einer Nukleinsäure, die für ein Tumor-assoziiertes Antigen oder einen Teil davon kodiert, der mindestens 6 aufeinanderfolgende Aminosäuren des wie in (a) definierten Tumor-assoziierten Antigens umfasst,
(iii) einer Antisense-Nukleinsäure, die spezifisch mit einer Nukleinsäure, die für ein Tumor-assoziiertes Antigen kodiert, hybridisiert,
(iv) einer Wirtszelle, die ein Tumor-assoziiertes Antigen oder einen Teil davon exprimiert, der mindestens 6 aufeinanderfolgende Aminosäuren des wie in (a) definierten Tumor-assoziierten Antigens umfasst, und
(v) isolierten Komplexen zwischen einem Tumor-assoziierten Antigen oder einem Teil davon und einem HLA-Molekül, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

In einem anderen Aspekt betrifft die Erfindung eine pharmazeutische Zusammensetzung umfassend einen Antikörper, der an die Aminosäuresequenz gemäß SEQ ID NO: 4 des Sequenzprotokolls bindet.

Das in Beispiel 3 näher beschriebene Tumor-assoziierte Antigen, das von einer Nukleinsäure gemäß SEQ ID NO: 3 kodiert wird, war im Stand der Technik nicht bekannt. WO02/12343 beschreibt lediglich die als SEQ ID NO: 33 bezeichnete Nukleinsäuresequenz, die für ein Protein kodiert, dessen 233 N-terminalen Aminosäuren identisch sind mit den 233 N-terminalen Aminosäuren des Tumor-assoziierten Antigens gemäß SEQ ID NO: 4 dieser Erfindung. Eine Nukleinsäuresequenz ("SEQ ID NO: 295"), die für ein Protein kodiert, dessen 233 N-terminalen Aminosäuren identisch sind mit den 233 N-terminalen Aminosäuren des Tumor-assoziierten Antigens gemäß SEQ ID NO: 4 dieser Erfindung, wird auch in CA2418130 beschrieben.

Eine Nukleinsäure, die für das erfindungsgemäß identifizierte Tumor-assoziierte Antigen oder einen Teil davon kodiert, kann in der pharmazeutische Zusammensetzung in einem Expressionsvektor vorliegen und funktionell mit einem Promotor verbunden sein.

Eine in der pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. Die Wirtszelle kann das HLA-Molekül endogen exprimieren. Alternativ kann die Wirtszelle das HLA-Molekül und/oder das Tumor-assoziierte Antigen oder den Teil davon rekombinant exprimieren. Die Wirtszelle kann weiterhin nicht-proliferativ sein, eine Antigen-präsentierende Zelle, eine dendritische Zelle, ein Monozyt oder ein Makrophage sein.

Ein in der pharmazeutischen Zusammensetzung enthaltener Antikörper kann ein monoklonaler Antikörper sein. In weiteren Ausführungsformen ist der Antikörper ein chimärer oder humanisierter Antikörper oder ein Fragment eines natürlichen Antikörpers, die durch kombinatorische Techniken hergestellt werden können. Der Antikörper kann mit einem therapeutisch oder diagnostisch nützlichen Mittel gekoppelt sein.

Eine in der pharmazeutischen Zusammensetzung enthaltene Antisense-Nukleinsäure kann eine Sequenz von 6-50, z.B. 10-30. 15-30 oder 20-30 zusammenhängenden Nukleotiden aus der Nukleinsäure, die für das erfindungsgemäß identifizierte Tumor-assoziierte Antigen kodiert, umfassen.

Ein durch die pharmazeutische Zusammensetzung entweder direkt oder durch die Expression einer Nukleinsäure bereitgestelltes Tumor-assoziiertes Antigen oder ein Teil davon kann an MHC-Moleküle auf der Oberfläche von Zellen binden, wobei die Bindung eine cytolytische Reaktion hervorrufen kann und/oder eine Zytokinausschüttung induzieren kann.

Die pharmazeutische Zusammensetzung kann einen pharmazeutisch verträglichen Träger und/oder ein Adjuvans umfassen. Das Adjuvans kann aus Saponin, GM-CSF, CpG-Oligonukleotiden, RNA, einem Zytokin oder einem Chemokin ausgewählt sein. Die pharmazeutische Zusammensetzung wird vorzugsweise zur Behandlung einer Erkrankung eingesetzt, die sich durch die Expression oder abnormale Expression eines Tumor-assoziierten Antigens auszeichnet. In einer bevorzugten Ausführungsform ist die Erkrankung Krebs.

Des weiteren betrifft die Erfindung Verfahren zur Diagnose oder Überwachung, d.h. Bestimmung der Regression, des Verlaufs und/oder des Ausbruchs, einer Erkrankung, die sich durch die Expression oder abnormale Expression des Tumorassoziierten Antigens auszeichnet.

Das erfindungsgemäße Verfahren zur Diagnose einer Erkrankung, die sich durch die Expression oder abnormale Expression eines Tumor-assoziierten Antigens auszeichnet, umfasst
(i) den Nachweis einer Nukleinsäure, die für das Tumor-assoziierte Antigen kodiert, oder eines Teils davon, der mindestens 6 aufeinanderfolgende Aminosäuren des wie in (a) definierten Tumor-assoziierten Antigens umfasst, und/oder
(ii) den Nachweis des Tumor-assoziierten Antigens oder eines Teils davon, der mindestens 6 aufeinanderfolgende Aminosäuren des wie in (a) definierten Tumor-assoziierten Antigens umfasst, und/oder
(iii) den Nachweis eines Antikörpers, der an die Aminosäuresequenz gemäß SEQ ID NO: 4 des Sequenzprotokolls bindet, und/oder
(iv) den Nachweis von cytotoxischen oder Helfer-T-Lymphozyten, die für das Tumor-assoziierte Antigen oder einen Teil davon spezifisch sind, wobei der Teil mindestens 6 aufeinanderfolgende Aminosäuren de wie in (a) definierten Tumor-assoziierten Antigens umfasst,
   in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Das Verfahren umfasst somit (i) den Nachweis einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. Der Nachweis (i) kann die Kontaktierung der biologischen Probe mit einem Mittel umfassen, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den cytotoxischen oder Helfer-T-Lymphozyten. Die Erkrankung kann sich durch die Expression oder abnormale Expression mehrerer verschiedener Tumor-assoziierter Antigene auszeichnen und der Nachweis kann einen Nachweis mehrerer Nukleinsäuren umfassen, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind. Die isolierte biologische Probe aus dem Patienten kann mit einer vergleichbaren normalen biologischen Probe verglichen werden.

Das Diagnoseverfahren ermöglicht auch eine Nutzung des erfindungsgemäß identifizierten Tumor-assoziierten Antigens als prognostischen Marker, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird.

In einem weiteren Aspekt betrifft die Erfindung ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon, der mindestens 6 aufeinanderfolgende Aminosäuren des wie in (a) definierten Tumor-assoziierten Antigens umfasst,
(iii) der Menge an Antikörpern, die an die Aminosäuresequenz gemäß SEQ ID NO: 4 des Sequenzprotokolls binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Die Überwachung kann eine Überwachung (i) der Menge mehrerer Nukleinsäuren umfassen, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder des Teils davon oder eine Bestimmung bzw. Überwachung der Menge einer Nukleinsäure oder des Teils davon kann mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. Die Polynukleotid-Sonde kann eine Sequenz von 6-50, also z.B. 10-30. 15-30 oder 20-30 zusammenhängenden Nukleotiden aus der Nukleinsäure umfassen.

Im Rahmen des Diagnoseverfahrens kann eine selektive Amplifikation des Promotorbereichs erfolgen oder eines Teils einer Nukleinsäure, die für das Tumor-assoziiertes Antigen kodiert und in Form von genomischer DNA vorliegt, nach Behandlung mit einem bisulfithaltigen Reagenz. Die Nukleinsäure wird vor einer Behandlung mit dem bisulfithaltigen Reagenz aus einer Probe eines zu untersuchenden Patienten isoliert. Die bei einer solchen Amplifikation verwendeten Oligonukleotide weisen z.B. eine Sequenz auf, die an die mit dem bisulfithaltigen Reagenz behandelte Nukleinsäure bindet, oder dazu vollständig komplementär ist.

Ein Nachweis des Tumor-assoziierten Antigens oder des Teils davon oder eine Bestimmung bzw. Überwachung der Menge des Tumor-assoziierten Antigens oder des Teils davon kann mit einem Antikörper erfolgen, der spezifisch an das Tumor-assoziierte Antigen oder den Teil davon bindet.

Das nachzuweisende Tumor-assoziierte Antigen oder der Teil davon können in einem Komplex mit einem MHC-Molekül, insbesondere einem HLA-Molekül vorliegen.

Ein Nachweis eines Antikörpers oder die Bestimmung bzw. Überwachung der Menge an Antikörpern kann mit einem Protein oder Peptid erfolgen, das spezifisch an den Antikörper bindet.

Ein Nachweis von cytolytischen T-Zellen oder Helfer-T-Zellen oder die Bestimmung bzw. Überwachung der Menge an cytolytischen T-Zellen oder Helfer-T-Zellen, die für Komplexe zwischen dem Antigen oder dem Teil davon und MHC-Molekülen spezifisch sind, kann mit einer Zelle erfolgen, die den Komplex zwischen dem Antigen oder dem Teil davon und einem MHC-Molekül präsentiert.

Die für einen Nachweis oder für eine Bestimmung bzw. Überwachung verwendete Polynukleotid-Sonde, der Antikörper, das Protein oder Peptid oder die Zelle sind z.B. nachweisbar markiert. Der nachweisbare Marker ist z.B. ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen

Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, der spezifische T-Lymphozyten identifizieren kann.

Die Erfindung betrifft auch Cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren,
wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
   und
(b) einer Nukleinsäure, die zu der Nukleinsäure unter (a) mindestens 90% Sequenzidentität aufweist.

Die Wirtszelle kann ein HLA-Molekül endogen exprimieren, oder sie exprimiert ein HLA-Molekül und/oder das Tumor-assoziierte Antigen oder den Teil davon rekombinant. Vorzugsweise ist die Wirtszelle nicht-proliferativ. Die Wirtszelle ist z.B. eine Antigen-präsentierende Zelle, insbesondere eine dendritische Zelle, ein Monozyt oder ein Makrophage.

Des weiteren betrifft die vorliegende Erfindung eine Nukleinsäure, die für ein Protein oder Polypeptid kodiert, das eine Aminosäuresequenz umfasst, die aus der Gruppe ausgewählt ist, bestehend aus SEQ ID NOs: 4, 7 und 8 des Sequenzprotokolls.

Ein rekombinantes Nukleinsäuremolekül, insbesondere DNA- oder RNA-Molekül, kann eine erfindungsgemäße Nukleinsäure umfassen.

Die Erfindung betrifft auch Wirtszellen, die eine erfindungsgemäße Nukleinsäure umfassen.

Die Wirtszelle kann ferner eine Nukleinsäure umfassen, die für ein HLA-Molekül kodiert, das z.B. endogen exprimiert wird. Alternativ exprimiert die Wirtszelle das HLA-Molekül und/oder die erfindungsgemäße Nukleinsäure oder einen Teil davon rekombinant. Die Wirtszelle kann nicht-proliferativ sein. Weiterhin kann die Wirtszelle eine

Antigen-präsentierende Zelle, insbesondere eine dendritische Zelle, ein Monozyt oder ein Makrophage sein.

Vorliegend werden auch Oligonulkeotid beschriebung, die mit einer erfindungsgemäß identifizierten Nukleinsäure hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft Bedingungen, die eine spezifische Hybridisierung zwischen Polynukleotiden erlauben.

In einem weiteren Aspekt betrifft die Erfindung ein Protein oder Polypeptid, das von einer erfindungsgemäßen Nukleinsäure kodiert wird. In einer bevorzugten Ausführungsform betrifft die Erfindung ein Protein oder Polypeptid, das eine Aminosäuresequenz umfasst, ausgewählt aus der Gruppe, bestehend aus SEQ ID NO: 4,7 und 8,

Vorliegend wird auch ein immunogenes Fragment des erfindungsgemäß identifizierten Tumor-assoziierten Antigens beschrieben. Das Fragment bindet z.B. an einen menschlichen HLA-Rezeptor oder menschlichen Antikörper. Beispielsweise umfasst ein solches Fragment eine Sequenz von mindestens 6, mindestens 8, mindestens 10, mindestens 12, mindestens 15, mindestens 20, mindestens 30 oder mindestens 50 Aminosäuren.

In einem weiteren Aspekt betrifft die Erfindung einen Antikörper, der an das erfindungsgemäß identifiziertes Tumor-assoziiertes Antigen oder an einen Teil davon bindet. Dieser Antikörper ist ein Antikörper, der spezifisch an die Aminosäuresequenz gemäß SEQ ID NO: 4 des Sequenzprotokolls bindet. Beispielsweise ist der Antikörper ein chimärer, ein humanisierter oder mit kombinatorischen Techniken hergestellte Antikörper oder ein Fragment eines Antikörpers.

Ein Antikörper kann ein monoklonaler Antikörper sein, ein chimärer oder humanisierter Antikörper oder ein Fragment eines natürlichen Antikörpers.

Des weiteren kann ein Antikörper mit einem therapeutischen oder diagnostischen Mittel gekoppelt sein. In einer Ausführungsform ist das therapeutische oder diagnostische Mittel ein Toxin.

In einem weiteren Aspekt betrifft die Erfindung einen Kit zum Nachweis der Expression oder abnormalen Expression eines Tumor-assoziierten Antigens, umfassend Mittel zum Nachweis
(i) der Nukleinsäure, die für das Tumor-assoziierte Antigen kodiert, oder eines Teils davon, der mindestens 6 aufeinanderfolgende Aminosäuren des wie in (a) definierten Tumor-assoziierten Antigens umfasst,
(ii) des Tumor-assoziierten Antigens oder eines Teils davon, der mindestens 6 aufeinanderfolgende Aminosäuren des wie in (a) definierten Tumor-assoziierten Antigens umfasst,
(iii) von Antikörpern, die an die Aminosäuresequenz gemäß SEQ ID NO: 4 des Sequenzprotokolls binden, und/oder
(iv) von T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Beispielsweise sind die Mittel zum Nachweis der Nukleinsäure oder des Teils davon Nukleinsäuremoleküle für die selektive Amplifikation der Nukleinsäure, die z.B. eine Sequenz von 6-50, z.B. 10-30, 15-30 oder 20-30 zusammenhängenden Nukleotiden aus der Nukleinsäure umfassen.

Die Erfindung betrifft insbesondere folgendes:

Die Erfindung betrifft in einem Aspekt eine pharmazeutische Zusammensetzung umfassend einen oder mehrere Bestandteile, die aus der Gruppe ausgewählt sind, bestehend aus:
(i) einem Tumor-assoziierten Antigen oder einem Teil davon, der mindestens 6 aufeinanderfolgende Aminosäuren des wie in (a) definierten Tumor-assoziierten Antigens umfasst,
(ii) einer Nukleinsäure, die für ein Tumor-assoziiertes Antigen oder einen Teil davon kodiert, der mindestens 6 aufeinanderfolgende Aminosäuren des wie in (a) definierten Tumor-assoziierten Antigens umfasst,
(iii) einer Antisense-Nukleinsäure, die spezifisch mit einer Nukleinsäure, die für ein Tumor-assoziiertes Antigen kodiert, hybridisiert,
(iv) einer Wirtszelle, die ein Tumor-assoziiertes Antigen oder einen Teil davon exprimiert, der mindestens 6 aufeinanderfolgende Aminosäuren des wie in (a) definierten Tumor-assoziierten Antigens umfasst, und
(v) isolierten Komplexen zwischen einem Tumor-assoziierten Antigen oder einem Teil davon und einem HLA-Molekül, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
   und
(b) einer Nukleinsäure, die zu der Nukleinsäure unter (a) mindestens 90% Sequenzidentität aufweist.

Die Erfindung betrifft in einem weiteren Aspekt eine pharmazeutische Zusammensetzung umfassend einen Antikörper, der an die Aminosäuresequenz gemäß SEQ ID NO: 4 des Sequenzprotokolls bindet.

In einer Ausführungsform ist die Wirtszelle eine Zelle, die das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

In einer weiteren Ausführungsform ist die pharmazeutische Zusammensetzung zur Behandlung einer Erkrankung vorgesehen, die sich durch die Expression oder abnormale Expression eines Tumor-assoziierten Antigens auszeichnet.

Die Erfindung betrifft weiterhin ein Verfahren zur Diagnose einer Erkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, oder eines Teils davon, der mindestens 6 aufeinanderfolgende Aminosäuren des wie in (a) definierten Tumor-assoziierten Antigens umfasst, und/oder
(ii) den Nachweis des Tumor-assoziierten Antigens oder eines Teils davon, der mindestens 6 aufeinanderfolgende Aminosäuren des wie in (a) definierten Tumor-assoziierten Antigens umfasst, und/oder
(iii) den Nachweis eines Antikörpers, der an die Aminosäuresequenz gemäß SEQ ID NO: 4 des Sequenzprotokolls bindet, und/oder
(iv) den Nachweis von cytotoxischen oder Helfer-T-Lymphozyten, die für das Tumor-assoziierte Antigen oder einen Teil davon spezifisch sind, wobei der Teil mindestens 6 aufeinanderfolgende Aminosäuren des wie in (a) definierten Tumor-assoziierten Antigens umfasst,
in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Die Erfindung betrifft weiterhin ein Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon, der mindestens 6 aufeinanderfolgende Aminosäuren des wie in (a) definierten Tumor-assoziierten Antigens umfasst,
(iii) der Menge an Antikörpern, die an die Aminosäuresequenz gemäß SEQ ID NO: 4 des Sequenzprotokolls binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Die Erfindung betrifft weiterhin einen Antikörper, der spezifisch an die Aminosäuresequenz gemäß SEQ ID NO: 4 des Sequenzprotokolls bindet.

In einer Ausführungsform ist der erfindungsgemäße Antikörper mit einem therapeutischen oder diagnostischen Mittel gekoppelt.

In einer weiteren Ausführungsform ist der erfindungsgemäße Antikörper zur Verwendung in einem Verfahren zur Behandlung, Diagnose oder Überwachung einer Erkrankung vorgesehen, wobei sich diese Erkrankung durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
   und
(b) einer Nukleinsäure, die zu der Nukleinsäure unter (a) mindestens 90% Sequenzidentität aufweist.

In einer weiteren Ausführungsform ist der erfindungsgemäße Antikörper zur Verwendung in einem Verfahren zur Behandlung, Diagnose oder Überwachung eines metastasierenden Tumors vorgesehen, wobei sich dieser Tumor durch die Expression oder abnormale Expression eines Tumorantigens auszeichnet, wobei das Verfahren die Verabreichung des Antikörpers umfasst, der Antikörper an das Tumorantigen oder einen Teil davon bindet und das Tumorantigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 23 des Sequenzprotokolls umfasst,
   und
(b) einer Nukleinsäure, die zu der Nukleinsäure unter (a) mindestens 90% Sequenzidentität aufweist.

In einer weiteren Ausführungsform ist der erfindungsgemäße Antikörper ein monoklonaler, chimärer oder humanisierter Antikörper oder ein Fragment eines Antikörpers.

In einem weiteren Aspekt betrifft die vorliegende Erfindung auch cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren,
wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
   und
(b) einer Nukleinsäure, die zu der Nukleinsäure unter (a) mindestens 90% Sequenzidentität aufweist.

In einer Ausführungsform exprimiert diese Wirtszelle ein HLA-Molekül rekombinant oder endogen, wobei das HLA-Molekül an das Tumor-assoziierte Antigen oder einen Teil davon bindet.

In einer weiteren Ausführungsform ist die erfindungsgemäße Erkrankung Krebs, insbesondere ein Colon-, Rektal-, Nieren-, Brust-, Prostata-, Gebärmutter-, Ovarial-, Endometrial-, Speiseröhren-, Blut-, Leber-, Pankreas-, Haut-, Gehirn- oder Lungenkrebs, ein Lymphom oder Neuroblastom, ein Lungen-, Brust-, Prostata-, HNO-, Magen-, Colontumor, Nierenzell-, Zervix-, Colon- oder Mammakarzinom oder Metastasen der vorstehenden Krebsarten oder Tumore.

In einer weiteren Ausführungsform umfasst das erfindungsgemäße Protein oder Polypeptid oder das Tumor-assoziierte Antigen eine Aminosäuresequenz, die aus der Gruppe ausgewählt ist, bestehend aus SEQ ID NOs: 4, 7 und 8 des Sequenzprotokolls.

Die Erfindung betrifft in einem weiteren Aspekt eine Nukleinsäure, die für ein Protein oder Polypeptid kodiert, das eine Aminosäuresequenz umfasst, die aus der Gruppe ausgewählt ist, bestehend aus SEQ ID NOs: 4, 7 und 8 des Sequenzprotokolls.

In einer Ausführungsform ist die erfindungsgemäße Nukleinsäure, eine Nukleinsäure, die eine Nukleinsäuresequenz gemäß SEQ ID NO: 3 des Sequenzprotokolls umfasst oder eine Nukleinsäure, die zu dieser Nukleinsäure mindestens 90% Sequenzidentität aufweist.

Die Erfindung betrifft in einem weiteren Aspekt eine Wirtszelle, die eine erfindungsgemäße Nukleinsäure umfasst.

Die Erfindung betrifft in einem weiteren Aspekt ein Protein oder Polypeptid, das von einer erfindungsgemäße Nukleinsäure kodiert wird. Die Erfindung betrifft in einem weiteren Aspekt ein Protein oder Polypeptid, das eine Aminosäuresequenz umfasst, die aus der Gruppe ausgewählt ist, bestehend aus SEQ ID NOs: 4, 7 und 8 des Sequenzprotokolls.

Die Erfindung betrifft in einem weiteren Aspekt einen Kit zum Nachweis der Expression oder abnormalen Expression eines Tumor-assoziierten Antigens, umfassend Mittel zum Nachweis
(i) der Nukleinsäure, die für das Tumor-assoziierte Antigen kodiert, oder eines Teils davon, der mindestens 6 aufeinanderfolgende Aminosäuren des wie in (a) definierten Tumor-assoziierten Antigens umfasst,
(ii) des Tumor-assoziierten Antigens oder eines Teils davon, der mindestens 6 aufeinanderfolgende Aminosäuren des wie in (a) definierten Tumor-assoziierten Antigens umfasst,
(iii) von Antikörpern, die an die Aminosäuresequenz gemäß SEQ ID NO: 4 des Sequenzprotokolls binden, und/oder
(iv) von T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst, und
(b) einer Nukleinsäure, die zu der Nukleinsäure unter (a) mindestens 90% Sequenzidentität aufweist.

Die Erfindung betrifft in einem weiteren Aspekt eine pharmazeutische Zusammensetzung, die RNA Interferenz (RNAi) umfasst, die die Expression oder Aktivität des Tumorantigens gemäß SEQ ID NO: 4 des Sequenzprotokolls hemmt.

In einer erfindungsgemäßen Ausführungsform enthält die RNAi eine short hairpin Struktur (shRNA).

### Detaillierte Beschreibung der Erfindung

Erfindungsgemäß wird ein Gene beschrieben, das in Tumorzellen selektiv exprimiert oder aberrant exprimiert wird und ein Tumor-assoziiertes Antigene darstellt.

Erfindungsgemäß sind Tumor-assoziierte Antigene bevorzugte Zielstrukturen für therapeutische Ansätze. Konzeptionell können die therapeutischen Ansätze auf eine Hemmung der Aktivität des selektiv exprimierten Tumor-assoziierten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist zum Beispiel Desoxyribonukleinsäure (DNA) oder Ribonukleinsäure (RNA). Nukleinsäuren umfassen genomische DNA, cDNA, mRNA, rekombinant hergestellte und chemisch synthetisierte Moleküle. Eine Nukleinsäure kann als einzelsträngiges oder doppelsträngiges und lineares oder kovalent kreisförmig geschlossenes Molekül vorliegen.

Die erfindungsgemäß beschriebenen Nukleinsäuren sind z.B. isoliert. Der Begriff "isolierte Nukleinsäure" bedeutet, dass die Nukleinsäure (i) in vitro amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC / 0,1 x SDS bei Temperaturen bis 68°C gewaschen.

Komplementäre Nukleinsäuren weisen z.B. mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können alleine oder in Kombination mit anderen Nukleinsäuren, insbesondere heterologen Nukleinsäuren, vorliegen. In einzelnen Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst Promotoren, Enhancer und andere Kontrollelemente, die die Expression eines Gens steuern. In bestimmten Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt Regulatorische Sequenzen können auch Enhancer-Sequenzen oder stromaufwärts gelegene Aktivatorsequenzen umfassen.

Zum einen kann also das hier dargestellte Tumorassoziierte Antigen mit beliebigen Expressionskontrollsequenzen und Promotoren kombiniert werden.

Zum anderen kann eine Nukleinsäure in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

Ein rekombinantes DNA-Molekül kann ein Vektor sein, gegebenenfalls mit einem Promotor, der die Expression einer Nukleinsäure, z.B. einer Nukleinsäure, die für das erfindungsgemäße Tumor-assoziierte Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden auch in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide, Bacteriophage oder Virusgenome.

Die Nukleinsäuren, die für das erfindungsgemäß identifizierte Tumor-assoziierte Antigen kodieren, können für eine Transfektion von Wirtszellen eingesetzt werden. Mit Nukleinsäuren ist dabei sowohl rekombinante DNA wie auch RNA gemeint. Rekombinante RNA kann durch *in vitro*-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen z.B. eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann ein Duplexmolekül mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Antisense-Oligonukleotide weisen z.B. eine Sequenz von 6-50, 10-30, 15-30 oder 20-30 zusammenhängenden Nukleotiden aus der Ziel-Nukleinsäure auf und sind z.B. auch vollständig zu der Ziel-Nukleinsäure oder einem Teil davon komplementär.

In denkbaren Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-SpleißStelle.

In einer Ausführungsform besteht ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In weiteren denkbaren Ausführungsformen ist ein Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens kann eine funktionelle Eigenschaft des Polypeptids auf weisen, dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Beispielsweise umfasst ein Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist beispielsweise ein Teil des Tumor-assoziierten Antigens, der dem nicht-Transmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft auch den Teil der Nukleinsäure, der zumindest für das Tumor-assoziierte Antigen und/oder für einen Teil oder ein Fragment des Tumor-assoziierten Antigens wie vorstehend definiert kodiert. Auch ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Es werden auch in bestimmten Ausführungsformen "dominant negative" Polypeptide beschrieben, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Es werden auch Stoffe wie Polypeptide beschrieben, die an das Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch
für eine Hemmung der Aktivität Tumor-assoziierter Antigene beispielsweise durch Bindung an solche Antigene Verwendung finden.

Daher sind z.B. Antikörper oder Antikörperfragment von interesse, die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet

Von Interesse sind auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1-und/oder CDR2-Regionen durch homologe menschliche oder nicht-menschliche Sequenzen ersetzt wurden sowie sogenannte einzelkettige Antikörper.

Ein erfindungsgemäß verwendeter Antikörper kahn z.B. durch Immunisierung mit Peptiden gemäß SEQ ID NOs: 4. 7 oder 8 erhalten werden.

Die vorstehend beschriebenen Antikörper und andere Bindemoleküle können beispielsweise für die Identifizierung von Gewebe verwendet werden, das ein Tumor-assozüertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluor und Gadolinium. Der Begriff "therapeutisch nützlicher Stoff' meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausfiihrungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Colon-, Rektal-, Nieren-, Brust-, Prostata-, Gebärmutter-, Ovarial-, Endometrial-, Speiseröhren-, Blut-, Leber-, Pankreas-, Haut-, Gehirn- und Lungenkrebs, Lymphome und Neuroblastome. Beispiele hierfür sind Lungen-, Brust-, Prostata-, Colontumor, Nierenzell-, Zervix-, Colon- und Mammakarzinom oder Metastasen der vorstehenden Krebsarten oder Tumore.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, Helfer-T-Zelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer-T-Zellen, die ein Tumor-assozüertes Antigen erkennen, gewünscht ist, wird die immunreaktive Zelle mit einer Zelle, die ein Tumor-assozüertes Antigen exprimiert, unter Bedingungen in Kontakt gebracht, die eine Produktion, Differenzierung und/oder Selektion von cytolytischen sowie Helfer-T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-96/33265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung.

Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet Tetramere werden sodann mit cytotoxischen T-Lymphozyten wie peripherem Blut oder Lymphknoten in Kontakt gebracht Die Tetramere binden an cytotoxische T-Lymphozyten, die den Peptid-Antigen/MHC-Klasse I-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen), mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen), mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat. Immunol. 2:962-70, 2001 ; Kessels et al., Nat. Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigen-Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhanceisequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigens in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigenpräsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer-T-Zell-Antworten entstehen können. Eine effektive Immunantwort benötigt diese (Ossendorp et al., Immunol. Lett. 74:75-9, 2000; Ossendorp et al., J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc. Natl. Acad. Sci. USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J. Immunol. 167:787-96, 2001; Appella et al., Biomed. Pept. Proteins Nucleic Acids 1:177-84, 1995; Wentworth et al., Mol. Immunol. 32:603-12, 1995).

Die beschriebenen pharmazeutischen Zusammensetzungen können auch als Vakzine für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL,-6/IL-12 zeigte eine Induktion des IFN-gamma- und Th1-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpem für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. Die Verabreichung von Nukleinsäuren kann z.B. durch *ex vivo*-Verfahren erfolgen, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. denkbar ist auch eine Verabreichung von Nukleinsäuren *in vivo* durch die Verwendung von Vektoren wie Viren und zielgesteuerten Liposomen.

Denkbar ist auch die Verwendung eines viralen Vektors für die Verabreichung einer Nukleinsäure, die für ein Tumor-assoziiertes Antigen kodiert. Solche Vektoren sind z.B. aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen in vitro oder in vivo einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausfiihrungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt z.B. durch ein Lungenaerosol. Die Verabreichung von Antisense-Nukleinsäuren erfolgt z.B. durch langsame intravenöse Verabreichung.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharnazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, z.B. von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäße einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugängliche.

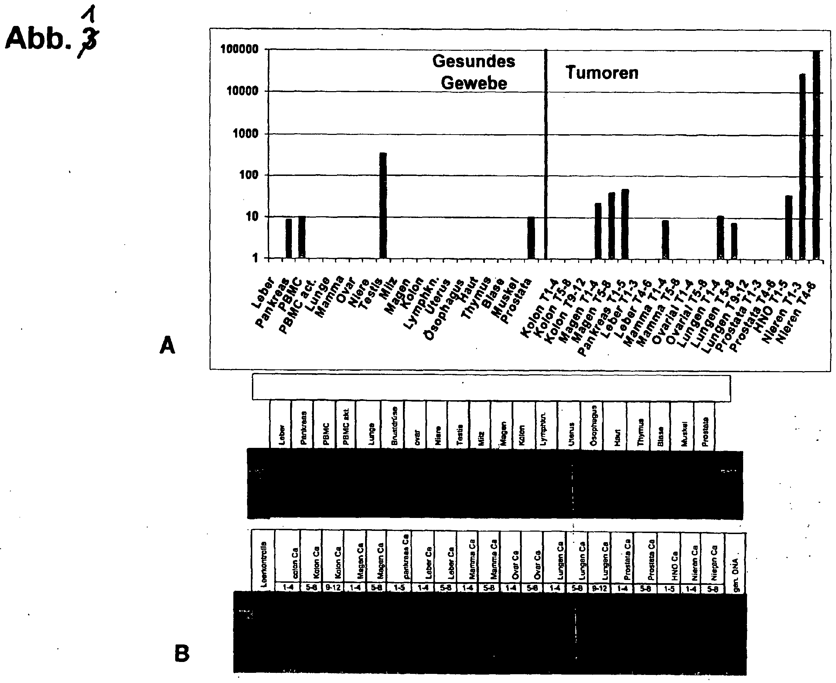

**Abb.1****: qPCR Analyse der SEQ ID NO: 1 in gesundem Gewebe und in Tumorenproben** Quantitative Expressionsanalyse der SEQ ID NO: 1 in Normalgeweben (linke Seite) und in verschiedenen Tumoren (Pools bestehend aus jeweils 3-5 Einzelproben, rechte Seite). **A** Logarithmische Darstellung der relativen Expression (fache Aktivierung). **B** Bild nach gelelektrophoretischer Auftrennung der amplifizierten Fragmente.

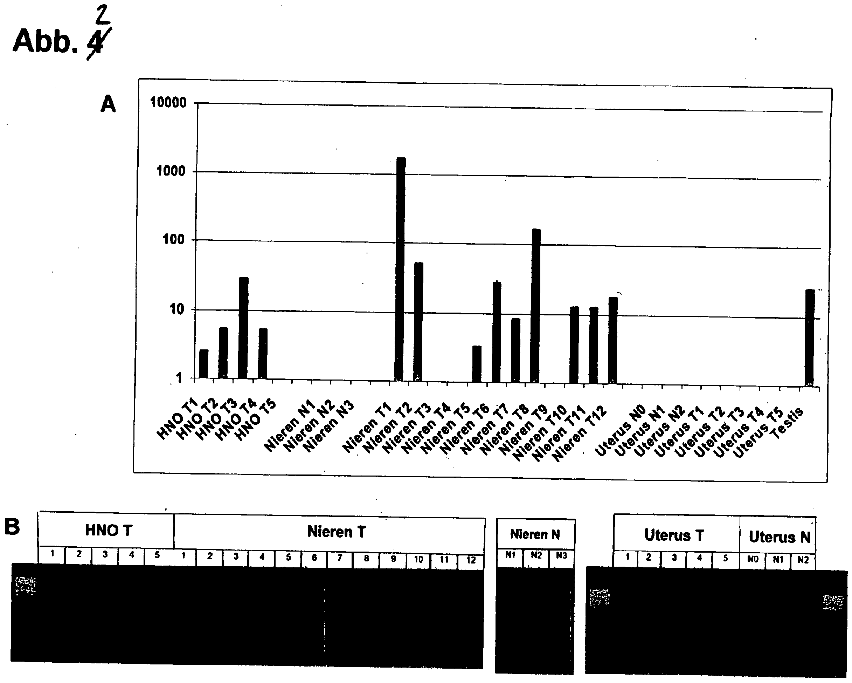

**Abb.2****: Detailanalyse der SEQ ID NO: 1-spezifischen Expression**
   A Quantitative Expressionsanalyse der SEQ ID NO: 1 in verschiedenen HNO-, Nieren- und Uterustumoren im Vergleich zur Expression in den zugehörigen Normalgeweben. Logarithmische Darstellung. **B** Bild nach gelelektrophoretischer Auftrennung der amplifizierten Fragmente.

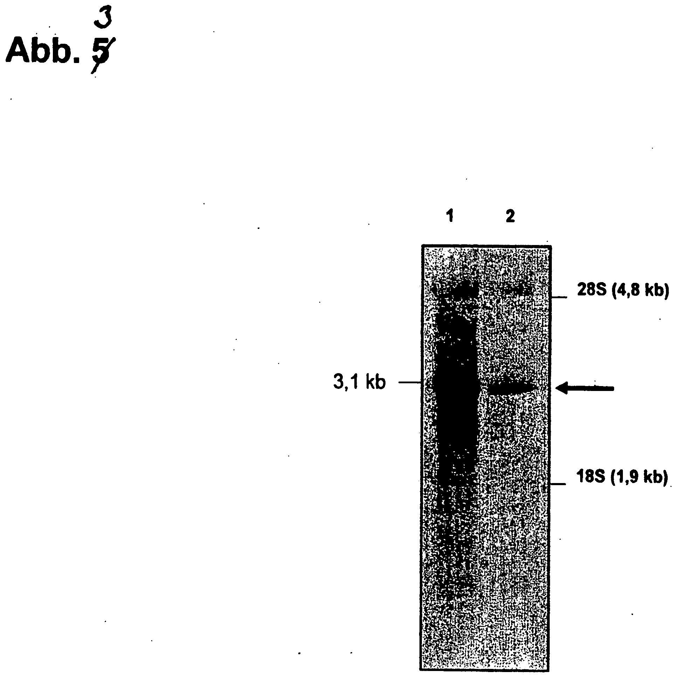

**Abb.3****: Northern blot Analyse mit einer SEQ ID NO: 1-spezifischen Sequenz** Hybridisierung einer DIG-markierten DNA-Sonde, die durch PCR-Amplifikation mit den Primern gemäß SEQ ID NO: 5 und 6 hergestellt wurde, mit Testis-spezifischer RNA. Spur 1: 2 µg Testis-spezifische RNA; Spur 2: 1 µg Testis-spezifische RNA

### Beispiele:

### Material und Methoden

Die Begriffe "*in silico"*, und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### Beispiel 1:

### Datamining-basierte Strategie zur Identifizierung von Tumor-assoziierten Antigenen

Erfindungsgemäß wurden öffentliche humane Protein- und Nukleinsäuredatenbanken im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildeten den zentralen Bestandteil dieser Strategie.

Den Ausgangspunkt bildeten die hauptsächlich aus dem humanen Genomprojekt vorhergesagten potenziellen Gene, die in der "RefSeq" Datenbank (Pruitt et al., Trends Genet. Jan;16(1):44-47, 2000) des "National Center for Biotechnology Information" (NCBI) als rein modellhafte Protein- (XP-) bzw. mRNA-Einträge (XM-) abgelegt sind. In einem weiteren Ansatz wurden auch die validierten Proteineinträge (NP-) bzw. die korrespondierenden mRNAs (NM-) derselben Datenbank in gleicher Weise analysiert. Dem Grundprinzip (hypothetisches) Gen zu mRNA zu Protein folgend wurden die Proteine unter Kombination mehrerer Prädiktionsprogramme für Proteinanalyse zunächst auf das Vorhanden sein von Transmembrandomänen hin untersucht. Aus der humanen XP-Fraktion der "RefSeq" Datenbank wurden insgesamt 19.544 Einträge analysiert, wobei 2.025 hypothetische Proteine den Filterkriterien genügten. Die humane NP-Fraktion lieferte insgesamt 19.110 Einträge mit einem Anteil von 4.634 gefilterten Proteinen.

Die korrespondierende mRNA jedes dieser 2.025 bzw. 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche stringent eingestellt. Insgesamt 1.270 hypothetische mRNAs erzielten dabei mindestens einen Treffer in der EST-Datenbank, wobei die Anzahl der Treffer in Einzelfällen mehr als 1.000 betrug.

Für jeden Einzelnen dieser validen Treffer wurde anschließend die gewebsspezifische Herkunft der zugrunde liegenden cDNA Bibliothek sowie der Name der Bibliothek ermittelt Die daraus resultierenden Gewebe wurden in vier verschiedene Gruppen eingeteilt, die von dispensiblen Organen (Gruppe 3) bis hin zu absolut lebensnotwendigen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST Treffer ausschließlich Krebsgewebe entstammten, gefolgt von denjenigen, die darüber hinaus noch in Geweben dispensibler Organe der Gruppe 3 zu finden sind.

Da es sich bei den im ersten Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit dem vorhergesagten Genlokus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu den erfindungsgemäßen Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### Beispiel 2:

### Validierungsstrategie der durch in silico Analyse identifizierten Tumor-assoziierten Antigene

Zur Nutzung der Targets für immuntherapeutische Zwecke (Antikörpertherapie mittels monoklonaler Antikörper, Vakzinierung, T-Zell Rezeptor-vermittelte therapeutische Ansätze; vgl. EP-B-0 879 282) oder andere zielgerichtete Ansätze (small compounds, siRNA etc.) bei der Krebstherapie sowie für diagnostische Fragestellungen ist die Validierung der erfindungsgemäß identifizierten Targets von zentraler Bedeutung. Die Validierung erfolgt dabei durch Expressionsanalyse sowohl auf RNA als auch auf Proteinebene.

### 1. Untersuchung der RNA Expression

Die erste Validierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittels Guanidiniumisothiocyanat wird die RNA mit saurem Phenol extrahiert, anschließend die RNA mit Isopropanol gefällt und in DEPC-behandeltes Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA Synthese erfolgt dabei mit Hilfe von zufälligen Hexameren (z.B. Roche Diagnostics) nach Standardprotokollen des jeweiligen Herstellers. Zur Qualitätskontrolle werden die cDNAs mit Primern in 30 Zyklen amplifiziert, die spezifisch für das nur gering exprimierte p53 Gen sind. Nur p53 positive cDNA Proben werden für die weiteren Reaktionsschritte verwendet.

Zur detaillierten Analyse der Targets wird auf Basis eines cDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polymerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionsbuffer (Endkonzentration 1 x, abhängig vom Hersteller der DNA-Polymerase) und je 0,3 mM des gen-spezifischen forward und reverse Primers.

Die spezifischen Primer des Zielgens werden, soweit möglich, so ausgewählt, das sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzyms zu aktivieren. Anschließend wird die DNA in 35 Zyklen amplifiziert (1 min 95°C, 1 min Primer spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative real time PCR erfolgen. Zu dieser Analyse sind inzwischen verschiedene Analysesysteme erhältlich, die bekanntesten sind das ABI PRISM Sequence detection system (TaqMan, Applied Biosystems), der iCycler (Biorad) sowie der Light cycler (Roche Diagnostics). Wie oben beschrieben wird ein spezifischer PCR Ansatz einem Lauf in den real time Geräten unterzogen. Durch Zusatz eines DNA interkalierenden Farbstoffes (z.B Ethidiumbromid, CybrGreen) wird die neu synthetisierte DNA durch spezifische Lichtanregung (nach Angaben der Farbstoffhersteller) sichtbar gemacht Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR Ansatzes erfolgt durch Messung eines "housekeeping Gens" (z.B. 18S RNA, β-Actin). Alternative Strategien über Fluoreszenz- markierte DNA-Sonden erlauben ebenfalls die quantitative Bestimmung des Zielgens aus einer spezifischen Gewebeprobe (siehe TaqMan Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Prädiktion des Proteins

Viele erfindungsgemäß gefundene Gene (insbesondere aus der XM Domäne der RefSeq) sind Gen-Neuentdeckungen, für die das Volllänge Gen kloniert, das offene Leseraster ermittelt und die Proteinsequenz abgeleitet und analysiert werden muss.
Für die Volllängeklonierung der Sequenz haben wir gängige Protokolle zur "Rapid amplification of cDNA ends", sowie Screening von cDNA Expressionsbanken mit genspezifischen Sonden verwendet *(*Sambrook et al., Molecular Cloning: A Laboratory Manual, 2. Auflage (1989), Cold Spring Harbor Laboratory Press, Cold Spring Harbor, N.Y.). Nach Zusammensetzung der so gefundenen Fragmente wurden potentielle Offene Leseraster (ORF) durch gängige Prädiktionsprogramme prädiziert. Da durch die Position des PolyA-Schwanzes und Polyadenylierungs-Motiven die Orientierung des potentiellen Genproduktes vorgegeben wird, verbleiben von möglichen 6 Leserastern nur noch die 3 der jeweiligen Orientierung. Oft ergibt sich aus diesen nur ein hinreichend großes offenes Leseraster, das für ein Protein kodieren kann, während die anderen Leseraster zu viele Stop-Codons aufweisen und für kein realistisches Proteine kodieren würden. Bei alternativen offenen Leserastern unterstützt die Berücksichtigung der Kozak-Kriterien für optimale Transkriptions-Initierung sowie die Analyse der sich potentiell ergebenden abgeleiteten Proteinsequenzen die Identifizierung des authentischen ORF. Dies wird weiter verifiziert durch Generierung von Immunseren gegen abgeleitete Proteine der potentiellen ORFs und ihre Analyse auf Erkennung eines realen Proteins in Geweben und Zelllinien.

### 4. Gewinnung von Antikörpern

Die Charakterisierung der erfindungsgemäß identifizierten Tumor-assoziierten Antigene erfolgt beispielsweise durch die Verwendung von Antikörpern. Ferner umfasst die Erfindung die diagnostische oder therapeutische Verwendung von Antikörpern. Dabei können Antikörper Proteine in nativem und/oder denaturierten Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von Antikörpern von Bedeutung, die therapeutisch eingesetzt werden sollen, aber auch für viele diagnostische Anwendungen. Dazu kann sowohl mit dem gesamten Protein als auch mit extrazellulären Teilsequenzen immunisiert werden.

### Immunisierung und Gewinnung von polyklonalen Antikörpern

Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der letzten Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonalen Antikörper, mit denen das Zielprotein im Western blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend).
(1) Im ersten Fall werden Peptide (Länge: 8-12 Aminosäuren) über *in vitro* Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung). Die Durchführung der Immunisierung kann auch als Service von Dienstleistern erfolgen.
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. E. *coli*), in Hefe (z.B. *S*. *pombe*), in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befinden sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Ziel gen wird exprimiert und das Tier entwickelt schließlich eine Immunantwort gegen das Zielprotein (Jung et al., Mol. Cells 12: 41-49, 2001; Kasinrerk et al., Hybrid Hybridomics 21: 287-293, 2002).

### Gewinnung monoklonaler Antikörper

Monoklonale Antikörper werden traditionell mit Hilfe der Hybridoma Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM" Technologie eingesetzt Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 5. Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Targetprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western blot (verschiedene Variationen sind z.B. In "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene mit transfiziert, um im folgenden Western blot die Spezifität des analysierten Antikörpers nachweisen zu können.

Im anschließenden Western blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elekrophoretisch aufgetrennt (SDS-Polyacrylamid Gelelektrophorese, SDS-PAGE). Anschließend werden die Proteine durch eines von mehreren Blotting Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im in silico Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das in die Zellen transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red, Dako) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden.

Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen synthetisieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60 minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristilierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glycosidischen Bindungen werden Proteinlysate vor der Denaturierung durch SDS mit O- oder N- Glykosylasen inkubiert (nach Angaben des jeweiligen Herstellers, z.B. PNgase, Endoglykosidase F, Endoglykosidase H, Roche Diagnostics). Anschließend erfolgt ein Western blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Targetmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of fünction") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA) Technologie in Zellen erzeugt werden (Hannon, GJ. 2002. RNA interference. Nature 418: 244-51; Czaudema et al. 2003. Nucl. Acid Res. 31: 670-82). Dazu werden Zellen mit kurzen, ca. 20-25 Nukleotide langen, doppelsträngigen RNA Molekülen transfiziert, die für das Zielmolekül spezifisch sind. Ein enzymatischer Prozess führt anschließend zum Abbau der spezifischen RNA des Zielgens und damit zu einer Funktionsinhibition des Zielproteins und ermöglicht damit die Analyse des Zielgens.

Zelllinien, die mittels Transfektion oder siRNA modifiziert wurden, können anschließend auf unterschiedliche Art und Weise analysiert werden. Nachstehend sind die geläufigsten Beispiele aufgeführt.

### 1. Proliferation und Zellzyklusverhalten

Eine Vielzahl von Verfahren sind zur Analyse der Zellproliferation etabliert und werden von verschiedenen Unternehmen kommerziell angeboten (z.B. Roche Diagnostics, Invitrogen, Details zu den Testverfahren sind in den zahlreichen Applikationsprotokollen beschrieben). Die Zellzahl in Zellkulturexperimenten lässt sich durch einfaches Auszählen oder durch kolometrische Tests ermitteln, die die metabolische Aktivität der Zellen messen (z.B. wst-1, Roche Diagnostics). Metabolische Testverfahren messen indirekt über enzymatische Marker die Zellzahl in einem Experiment. Direkt kann die Zellproliferation durch Analyse der DNA Syntheserate z.B. durch Zugabe von Bromdesoxyuridin (BrdU) gemessen werden, der Nachweis des integrierten BrdU erfolgt über spezifische Antikörper kolometrisch.

### 2. Apoptose und Zytotoxizität

Eine große Anzahl von Testsystemen zum Nachweis von zellulärer Apoptose und von Zytotoxizität sind verfügbar. Ein entscheidendes Charakteristikum ist die spezifische, enzymabhängige Fragmentierung der genomischen DNA, die irreversibel ist und sicher zum Tod der Zelle führt. Verfahren zum Nachweis dieser spezifischen DNA Fragmente sind kommerziell erhältlich. Als zusätzliches Verfahren steht der "TUNEL assay" zur Verfügung, der DNA Einzelstrangbrüche auch in Gewebeschnitten nachweisen kann. Zytotoxizität wird vor allem über eine veränderte Zellpermeabilität nachgewiesen, die als Marker für den Vitalitätszustand von Zellen dient Dazu werden entweder im Zellkulturüberstand Marker analysiert, die normalerweise intrazellulär zu finden sind. Alternativ kann auch die Aufnahmefähigkeit von Farbmarkern analysiert werden, die von intakten Zellen nicht aufgenommen werden. Die bekanntesten Beispiele für Farbmarker sind Trypanblau und Propidiumiodid, ein üblicher intrazellulärer Marker ist die Laktatdehydrogenase, die im Überstand enzymatisch nachgewiesen werden kann. Unterschiedliche Testsysteme stehen von verschiedenen kommerziellen Anbietern (z.B. Roche Diagnostics, Invitrogene) zur Verfügung.

### 3. Migrationsassay

Die Fähigkeit von Zellen zur Migration wird in einem spezifischen Migrationstest vorzugsweise mit Hilfe einer Boyden Kammer (Corning Costar) analysiert (Cinamon G., Alon R. J. Immunol. Methods. 2003 Feb; 273(1-2):53-62; Stockton et al. 2001. Mol. Biol. Cell. 12: 1937-56). Dazu werden Zellen auf einem Filter mit spezifischer Porengröße kultiviert. Zellen, die migrieren können, sind in der Lage, durch diesen Filter in ein weiteres darunter liegendes Kulturgefäß zu wandern. Eine anschließende mikroskopische Analyse erlaubt dann die Bestimmung eines möglicherweise veränderten Migrationsverhaltens, dass durch den "gain of function" bzw. "loss of function" des Zielmoleküls induziert wurde.

### b. Funktionsanalyse in Tiermodellen

Alternativ zu Zellkulturexperimenten bieten sich zur Analyse der Zielgenfunktion aufwendige in vivo Experimente in Tiermodellen an. Diese Modelle haben im Vergleich zu den zellbasierenden Verfahren den Vorteil, dass sie Fehlentwicklungen bzw. Krankheiten nachweisen können, die erst im Kontext des gesamten Organismus nachweisbar sind. Eine Vielzahl von Modellen für humane Erkrankungen sind inzwischen verfügbar (Abate-Shen & Shen. 2002. Trends in Genetics S1-5; Matsusue et. al. 2003. J. Clin. Invest. 111:737-47). Verschiedene Tiermodelle wie zum Beispiel Hefe, Nematoden oder Zebraüsche sind inzwischen intensiv charakterisiert worden. Bevorzugte Modelle sind aber im Vergleich zu anderen Spezies mammale Tiermodelle wie zum Beispiel die Maus (Mus musculus), weil sie die biologischen Prozesse im humanen Kontext am besten abbilden können. Für Mäuse sind in den letzten Jahren sowohl transgene Verfahren etabliert worden, die neue Gene in das Mausgenom integrieren ("gain of function"; Jegstrup I. et al. 2003. Lab Anim. 2003 Jan.;37(1):1-9). Alternativ werden durch andere methodische Ansätze Gene im Mausgenom ausgeschaltet und so ein Funktionsverlust eines gewünschten Gens induziert (knockout Modelle, "loss of function"; Zambrowicz BP & Sands AT. 2003. Nat. Rev. Drug Discov. 2003 Jan;2(1):38-51; Niwa H. 2001. Cell Struct. Funct. 2001 Jun;26(3):137-48.); technische Details sind vielfältig publiziert.

Nach Generierung der Mausmodelle können Veränderungen, die durch das Transgen bzw. durch den Funktionsverlust eines Gens induziert wurden, im Kontext des Gesamtorganismus analysiert werden (Balling R, 2001. Ann. Rev. Genomics Hum. Genet. 2:463-92). So sind zum Beispiel Verhaltenstests genauso wie biochemische Untersuchen etablierter Blutparameter möglich. Histologische Analysen, Immunhistochemie oder die Elektronenmikroskopie ermöglichen die Charakterisierung von Veränderungen auf zellulärer Ebene. Das spezifische Expressionsmuster eines Genes kann durch eine in situ Hybridisierung nachgewiesen werden (Peters T. et. al. 2003. Hum. Mol. Genet 12: 2109-20).

### Beispiel 3: Identifizierung der SEQ ID NO: 3/4 als diagnostisches und therapeutisches Krebs-Target

Die SEQ ID NO: 1 (Nukleinsäuresequenz) wird von einem neuen Gen auf Chromosom 11 (11q12.1) kodiert und repräsentiert die abgeleitete Proteinsequenz (SEQ ID NO: 2). Ein alternatives offenes Leseraster dieses Genlokus ist SEQ ID NO: 3 die für die abgeleitete Proteinsequenz SEQ ID NO: 4 kodiert. Beide Proteinsequenzen zeigen keine Homologien zu bereits bekannten Proteinen.

Erfindungsgemäß wurde nach der Etablierung einer genspezifischen quantitativen RT-PCR (Primerpaar SEQ ID NO: 5 und 6 die Menge der genspezifischen Transkript in gesundem Gewebe und in Karzinomproben (jeweils Pool von Proben) untersucht. Spezifische RNA detektierten wir mit Ausnahme von Testis gar nicht oder aber nur in geringen Mengen in den von uns untersuchten gesunden Geweben (Abb.1; A quantitative RT-PCR; B Gelbild). Der Lokus exprimiert demnach mit hoher Wahrscheinlichkeit ein keimzellspezifisches Genprodukt. Allerdings ist das Gen in vielen Tumorproben aktiviert, spezifische RNA war in substantiellen Mengen nachweisbar (Abb.1). Die höchste Prävalenz und Expressionshöhe fanden wir in Nierenzelltumoren. Aber auch in Magen-, Pankreas-, HNO- und Lungentumoren waren spezifische Transkripte nachweisbar (Abb.2; A quantitative RT-PCR; B Gelbild). Auch wiederholte Untersuchungen der korrespondierenden Normalgewebe waren gen-spezifische Transkripte nicht detektierbar. Um die Expression von diesem Genlokus zusätzlich zu belegen wurde zusätzlich ein Northern blot durchgeführt. Dazu wurde eine Sonde in einer spezifischen PCR der Primer SEQ ID NO: 5 und 6 unter Einbau von Digoxygenin-dUTP (Roche Diagnostics) nach Angaben des Herstellers hergestellt. Die Sonde wurde anschließend mit 2µg (Abb.3, Spur 1) bzw. 1 µg (Abb.3, Spur 2) Gesamt-RNA aus Testisgewebe hybridisiert und das Digoxygenin der Sonde anschließend in einer spezifischen Farbreaktion nachgewiesen. Ein ca. 3,1 kB großes, Gen-spezifisches Fragment konnte in dem Experiment nachgewiesen werden (Abb. 3), und bestätigte somit zusätzlich die Expression dieses Lokus.

Der Genlokus ist somit ein typischer Vertreter der Klasse der sog. Cancer/Testis-Antigene, die in Normalgeweben fast ausschließlich in den Keimzellen der Testis exprimiert sind. In Tumoren jedoch werden Cancer/Testis-Antigene häufig eingeschaltet, obwohl sie in den zugrunde liegenden somatischen Normalgewebszellen nicht exprimiert werden. Mehrere Mitglieder dieser funktionell und strukturell heterogenen Klasse werden aufgrund ihrer attraktiven selektiven Gewebsverteilung bereits für spezifische immuntherapeutischen Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).

Zur Herstellung von Antikörpern können die Peptide gemäß SEQ ID NO: 7 und 8 genutzt werden. Insbesondere die extrazellulären Domänen der SEQ ID NO: 2 bzw. SEQ ID NO: 4 können erfindungsgemäß als Zielstruktur von monoklonalen Antikörpern genutzt werden.

## Patentansprüche

1. Pharmazeutische Zusammensetzung umfassend einen oder mehrere Bestandteile, die aus der Gruppe ausgewählt sind, bestehend aus:
(i) einem Tumor-assoziierten Antigen oder einem Teil davon, der mindestens 6 aufeinanderfolgende Aminosäuren des Tumor-assoziierten Antigens umfasst,
(ii) einer Nukleinsäure, die für ein Tumor-assoziiertes Antigen oder einen Teil davon kodiert, der mindestens 6 aufeinanderfolgende Aminosäuren des des wie in (a) definierten Tumor-assoziierten Antigens umfasst,
(iii) einer Antisense-Nukleinsäure, die spezifisch mit einer Nukleinsäure, die für ein Tumor-assoziiertes Antigen kodiert, hybridisiert,
(iv) einer Wirtszelle, die ein Tumor-assoziiertes Antigen oder einen Teil davon exprimiert, der mindestens 6 aufeinanderfolgende Aminosäuren des des wie in (a) definierten **Tumor-assoziierten** Antigens umfasst, und
(v) isolierten Komplexen zwischen einem Tumor-assoziierten Antigen oder einem Teil davon und einem HLA-Molekül, wobei der Teil mindestens 6 aufeinanderfolgende Aminosäuren des des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

2. Pharmazeutische Zusammensetzung umfassend einen Antikörper, der an die Aminosäuresequenz gemäß SEQ ID NO: 4 des Sequenzprotokolls bindet.

3. Pharmazeutische Zusammensetzung nach Anspruch 1, wobei die Wirtszelle das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

4. Pharmazeutische Zusammensetzung nach einem der Ansprüche 1-3 zur Behandlung einer Erkrankung, die sich durch die Expression oder abnormale Expression eines Tumor-assoziierten Antigens auszeichnet.

5. Verfahren zur Diagnose einer Erkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, oder eines Teils davon, der mindestens 6 aufeinanderfolgende Aminosäuren des des wie in (a) definierten Tumor-assoziierten Antigens umfasst, und/oder
(ii) den Nachweis des Tumor-assoziierten Antigens oder eines Teils davon, der mindestens 6 aufeinanderfolgende Aminosäuren des des wie in (a) definierten Tumor-assoziierten Antigens umfasst, und/oder
(iii) den Nachweis eines Antikörpers, der an die Aminosäuresequenz gemäß SEQ ID NO: 4 des Sequenzprotokolls bindet, und/oder
(iv) den Nachweis von cytotoxischen oder Helfer-T-Lymphozyten, die für das Tumor-assoziierte Antigen oder einen Teil davon spezifisch sind, wobei der Teil mindestens 6 aufeinanderfolgende Aminosäuren des des wie in (a) definierten Tumor-assoziierten Antigens umfasst,
in einer aus einem Patienten isolierten biologischen Probe, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

6. Verfahren zur Bestimmung der Regression, des Verlaufs oder des Ausbruchs einer Erkrankung, die sich durch die expression oder abnormale expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon, der mindestens 6 aufeinanderfolgende Aminosäuren des des wie in (a) definierten Tumor-assoziierten Antigens umfasst,
(iii) der Menge an Antikörpern, die an die Aminosäuresequenz gemäß SEQ ID NO: 4 des Sequenzprotokolls binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des des wie in (a) definierten Tumor-assoziierten Antigens umfasst,
wobei das Tumor-assoziierte Antigen eine Sequenz ausweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokollsumfasst,
und
(b) einer Nukleinsäure, die zu der Nukleinsäure unter (a) mindestens 90% Sequenzidentität aufweist.

7. Antikörper, der spezifisch an die Aminosäuresequenz gemäß SEQ ID NO: 4 des Sequenzprotokolls bindet.

8. Pharmazeutische Zusammensetzung nach Anspruch 2 oder Antikörper nach Anspruch 7, wobei der Antikörper mit einem therapeutischen oder diagnostischen Mittel gekoppelt ist.

9. Antikörper nach Anspruch 8 zur Verwendung in einem Verfahren zur Behandlung, Diagnose oder Überwachung einer Erkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

10. Antikörper nach Anspruch 8 zur Verwendung in einem Verfahren zur Behandlung, Diagnose oder Überwachung eines metastasierenden Tumors, der sich durch die Expression oder abnormaler Expression eines Tumorantigens auszeichnet, wobei das Verfahren die Verabreichung des Antikörpers umfasst und das Tumorantigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

11. Pharmazeutische Zusammensetzung nach Anspruch 2 oder 8, Pharmazeutische Zusammensetzung zur Verwendung nach Anspruch 4, Antikörper nach Anspruch 7 oder 8 oder Antikörper zur Verwendung nach Anspruch 9 oder 10, wobei der Antikörper ein monoklonaler, chimärer oder humanisierter Antikörper oder ein Fragment eines Antikörpers ist.

12. Cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren,
wobei der Teil mindestens 6 aufeinanderfolgende Aminosäuren des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweiset.

13. Cytolytische oder Cytokine-ausschüttende T-Zellen nach Anspruch 12, wobei die Wirtszelle ein HLA-Molekül rekombinant oder endogen exprimiert, das an das Tumor-assoziierte Antigen oder einen Teil davon bindet.

14. Pharmazeutische Zusammensetzung nach Anspruch 4, Verfahren nach Anspruch 5 oder 6, Antikörper nach einem der Ansprüche 9 bis 11, oder cytolytische oder Cytokine-ausschüttende T-Zellen nach Anspruch 12 oder 13, wobei die Erkrankung Krebs ist.

15. Pharmazeutische Zusammensetzung nach Anspruch 4, Verfahren nach Anspruch 5 oder 6, Antikörper nach einem der Ansprüche 9 bis 11, oder cytolytische oder Cytokine-ausschüttende T-Zellen nach Anspruch 12 oder 13, wobei die Erkrankung ein Colon-, Rektal-, Nieren-, Brust-, Prostata-, Gebärmutter-, Ovarial-, Endometrial-, Speiseröhren-, Blut-, Leber-, Pankreas-, Haut-, Gehirn- oder Lungenkrebs, ein Lymphom oder Neuroblastom, ein Lungen-, Brust-, Prostata-, HNO-, Magen-, Colontumor, Nierenzell-, Zervix-, Colon- oder Mammakarzinom oder Metastasen der vorstehenden Krebsarten oder Tumore ist.

16. Pharmazeutische Zusammensetzung nach einem der Ansprüche 1 bis 4, 8, 9, 14 und 15, Verfahren nach einem der Ansprüche 5, 6, 14 und 15, Antikörper nach einem der Ansprüche 7 bis 11, 14 und 15, oder cytolytische oder Cytokine-ausschüttende T-Zellen nach einem der Ansprüche 12 bis 15, wobei das Protein oder Polypeptid oder das Tumor-assoziierte Antigen eine Aminosäuresequenz umfasst, die aus der Gruppe ausgewählt ist, bestehend aus SEQ ID NOs: 4,7 und 8 des Sequenzprotokolls.

17. Nukleinsäure, die für ein Protein oder Polypeptid kodiert, das eine Aminosäuresequenz umfasst, die aus der Gruppe ausgewählt ist, bestehend aus SEQ ID NOs: 4,7 und 8 des Sequenzprotokolls.

18. Nukleinsäure nach Anspruch 17, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

19. Wirtszelle, die eine Nukleinsäure nach Anspruch 17 oder 18 umfasst.

20. Protein oder Polypeptid, das von einer Nukleinsäure nach Anspruch 17 oder 18 kodiert wirt.

21. Protein oder Polypeptid, das eine Aminosäuresequenz umfasst, die aus der Gruppe ausgewählt ist, bestehend aus SEQ ID NOs: 4, 7 und 8 des Sequenzprotokolls.

22. Kit zum Nachweis der Expression oder abnormalen Expression eines Tumor-assoziierten Antigens, umfassend Mittel zum Nachweis
(i) der Nukleinsäure, die für das Tumor-assoziierte Antigen kodiert, oder eines Teils davon, der mindestens 6 aufeinanderfolgende Aminosäuren des des wie in (a) definierten Tumor-assöziierten Antigens umfasst,
(ii) des Tumor-assoziierten Antigens oder eines Teils davon, der mindestens 6 aufeinanderfolgende Aminosäuren des des wie in (a) definierten Tumor-assoziierten Antigens umfasst,
(iii) von Antikörpern, die an die Aminosäuresequenz gemäß SEQ ID NO: 4 des Sequenzprotokolls binden, und/oder
(iv) von T-Zellen, die für einen Komplex zwischen dem Tumor-assoziierten Antigen oder einem Teil davon und einem MHC-Molekül spezifisch sind, wobei der Teil mindestens 6 aufeinanderfolgende Aminosäuren des des wie in (a) definierten Tumor-assoziierten Antigens umfasst,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz gemäß SEQ ID NO: 3 des Sequenzprotokolls umfasst,
und
(b) einer Nukleinsäure, die zu der Nukleinsäure unter (a) mindestens 90% Sequenzidentität aufweist.

23. Pharmazeutische Zusammensetzung, die RNA Interferenz (RNAi) umfasst, die die Expression oder Aktivität des Tumorantigens gemäß SEQ ID NO: 4 des Sequenzprotokolls hemmt.

24. Pharmazeutische Zusammensetzung nach Anspruch 23, wobei die RNAi eine short hairpin Struktur (shRNA) enthält.

## Claims

1. A pharmaceutical composition, comprising one or more components selected from the group consisting of:
(i) a tumor-associated antigen, or a part thereof comprising at least 6 consecutive amino acids of the tumor-associated antigen,
(ii) a nucleic acid which codes for a tumor-associated antigen, or a part thereof comprising at least 6 consecutive amino acids of the tumor-associated antigen as defined under (a),
(iii) an antisense nucleic acid which specifically hybridizes with a nucleic acid encoding a tumor-associated antigen,
(iv) a host cell which expresses a tumor-associated antigen, or a part thereof comprising at least 6 consecutive amino acids of the tumor-associated antigen as defined under (a), and
(v) isolated complexes between a tumor-associated antigen or a part thereof and an HLA molecule, wherein the part comprises at least 6 consecutive amino acids of the tumor-associated antigen as defined under (a),
the tumor-associated antigen having a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

2. A pharmaceutical composition, comprising an antibody which binds to an amino acid sequence according to SEQ ID NO: 4 of the sequence listing.

3. The pharmaceutical composition of claim 1, wherein the host cell secretes the tumor-associated antigen or the part thereof, expresses it on the surface or expresses an HLA-molecule which binds to the tumor-associated antigen or to the part thereof.

4. The pharmaceutical composition of any one of claims 1-3 for the treatment of a disease **characterized by** expression or abnormal expression of a tumor-associated antigen.

5. A method for diagnosing a disease **characterized by** expression or abnormal expression of a tumor-associated antigen, comprising
(i) detection of a nucleic acid which codes for the tumor-associated antigen, or for a part thereof comprising at least 6 consecutive amino acids of the tumor-associated antigen as defined under (a), and/or
(ii) detection of the tumor-associated antigen, or of a part thereof comprising at least 6 consecutive amino acids of the tumor-associated antigen as defined under (a), and/or
(iii) detection of an antibody which binds to the amino acid sequence according to SEQ ID NO: 4 of the sequence listing, and/or
(iv) detection of cytotoxic or T helper lymphocytes which are specific to the tumor-associated antigen or to a part thereof, wherein the part comprises at least 6 consecutive amino acids of the tumor-associated antigen as defined under (a),
in a biological sample isolated from a patient,
the tumor-associated antigen having a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

6. A method for determining regression, course or onset of a disease **characterized by** expression or abnormal expression of a tumor-associated antigen, comprising monitoring a sample from a patient who has the disease or is suspected of falling ill with the disease, with respect to one or more parameters selected from the group consisting of:
(i) the amount of the nucleic acid which codes for the tumor-associated antigen or for a part thereof,
(ii) the amount of the tumor-associated antigen, or of a part thereof comprising at least 6 consecutive amino acids of the tumor associated antigen as defined under (a),
(iii) the amount of antibodies which bind to the amino acid sequence according to SEQ ID NO: 4 of the sequence listing, and
(iv) the amount of cytolytic or cytokine-releasing T cells which are specific for a complex between the tumor-associated antigen or a part thereof and an MHC molecule, wherein the part comprises at least 6 consecutive amino acids of the tumor-associated antigen as defined under (a),
the tumor-associated antigen having a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

7. An antibody which binds specifically to the amino acid sequence according to SEQ ID NO: 4 of the sequence listing.

8. The pharmaceutical composition of claim 2 or the antibody of claim 7, wherein the antibody is coupled to a therapeutic or diagnostic agent.

9. The antibody of claim 8 for use in a method of treating, diagnosing or monitoring a disease **characterized by** expression or abnormal expression of a tumor-associated antigen, wherein the method comprises the administration of the antibody and the tumor-associated antigen has a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

10. The antibody of claim 8 for use in a method of treating, diagnosing or monitoring a metastasizing tumor **characterized by** expression or abnormal expression of a tumor antigen, wherein the method comprises the administration of the antibody and the tumor antigen has a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

11. The pharmaceutical composition of claim 2 or 8, the pharmaceutical composition for use of claim 4, the antibody of claim 7 or 8 or the antibody for use of claim 9 or 10, wherein the antibody is a monoclonal, chimeric or humanized antibody or a fragment of an antibody.

12. Cytolytic or cytokine-releasing T cells for use in a method of treating a patient having a disease **characterized by** expression or abnormal expression of a tumor-associated antigen, comprising:
(i) removing a sample containing immunoreactive cells from the patient,
(ii) contacting the sample with a host cell expressing the tumor-associated antigen or a part thereof, under conditions which favor production of cytolytic or cytokine-releasing T cells against the tumor-associated antigen or a part thereof, and
(iii) introducing the cytolytic or cytokine-releasing T cells into the patient in an amount suitable for lysing cells expressing the tumor-associated antigen or a part thereof,
wherein the part comprises at least 6 consecutive amino acids of the tumor-associated antigen as defined under (a),
the tumor-associated antigen having a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

13. The cytolytic or cytokine-releasing T cells of claim 12, wherein the host cell recombinantly or endogenously expresses an HLA molecule binding to the tumor-associated antigen or to the part thereof.

14. The pharmaceutical composition of claim 4, the method of claim 5 or 6, the antibody of any one of claims 9 to 11, or the cytolytic or cytokine-releasing T-cells of claim 12 and 13, wherein the disease is cancer.

15. The pharmaceutical composition of claim 4, the method of claim 5 or 6, the antibody of any one of claims 9 to 11, or the cytolytic or cytokine-releasing T cells of claim 12 or 13, wherein the disease is a colon-, rectal-, kidney-, breast-, prostate-, uterus-, ovarial-, endometrial-, esophageal-, blood-, liver-, pancreas-, skin-, brain-, or lung cancer, a lymphoma or neuroblastoma, a lung-, breast-, prostate-, ENT-, stomach-, colon tumor, a renal cell-, cervical-, colon- or mammary carcinoma or metastases of the aforementioned cancer species or tumors.

16. The pharmaceutical composition of any one of claims 1 to 4, 8, 9, 14 and 15, the method of any one of claims 5, 6, 14 and 15, the antibody of any one of claims 7 to 11, 14 and 15, or the cytolytic or cytokine releasing T cells of any one of claims 12 to 15, wherein the protein or polypeptide or the tumor-associated antigen comprises an amino acid sequence selected from the group consisting of SEQ ID NOs: 4, 7 and 8 of the sequence listing.

17. Nucleic acid which codes for a protein or polypeptide comprising an amino acid sequence selected from the group consisting of SEQ ID NOs: 4, 7 and 8 of the sequence listing.

18. Nucleic acid of claim 17 selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

19. Host cell comprising a nucleic acid of claim 17 or 18.

20. Protein or polypeptide which is encoded by a nucleic acid of claim 17 or 18.

21. Protein or polypeptide comprising an amino acid sequence selected from the group consisting of SEQ ID NOs: 4, 7 and 8 of the sequence listing.

22. Kit for detecting expression or abnormal expression of a tumor-associated antigen, comprising agents for detection
(i) of the nucleic acid, which codes for the tumor-associated antigen, or a part thereof comprising at least 6 consecutive amino acids of the tumor-associated antigen as defined under (a),
(ii) of the tumor-associated antigen, or a part thereof comprising at least 6 consecutive amino acids of the tumor-associated antigen as defined under (a),
(iii) of antibodies which bind to the amino acid sequence according to SEQ ID NO: 4 of the sequence listing, and/or
(iv) of T cells which are specific for a complex between the tumor-associated antigen or a part thereof and an MHC molecule, the part comprising at least 6 consecutive amino acids of the tumor-associated antigen as defined under (a),
the tumor-associated antigen having a sequence encoded by a nucleic acid which is selected from the group consisting of:
(a) a nucleic acid which comprises a nucleic acid sequence according to SEQ ID NO: 3 of the sequence listing, and
(b) a nucleic acid which has at least 90% sequence identity to the nucleic acid of (a).

23. Pharmaceutical composition, comprising RNA interference (RNAi) inhibiting expression or activity of the tumor antigen according to SEQ ID NO: 4 of the sequence listing.

24. Pharmaceutical composition of claim 23, wherein the RNAi comprises a short hairpin structure (shRNA).

## Revendications

1. Composition pharmaceutique comprenant un ou plusieurs constituants qui sont choisis dans le groupe consistant en:
(i) un antigène associé à une tumeur ou une partie de celui-ci qui comprend au moins 6 acides aminés consécutifs de l'antigène associé à une tumeur,
(ii) un acide nucléique qui code pour un antigène associé à une tumeur ou une partie de celui-ci qui comprend au moins 6 acides aminés consécutifs de l'antigène associé à une tumeur tel que défini sous (a),
(iii) un acide nucléique antisens qui hybride spécifiquement avec un acide nucléique qui code pour un antigène associé à une tumeur,
(iv) une cellule hôte qui exprime un antigène associé à une tumeur ou une partie de celui-ci qui comprend au moins 6 acides aminés consécutifs de l'antigène associé à une tumeur tel que défini sous (a), et
(v) des complexes isolés entre un antigène associé à une tumeur ou une partie de celui-ci et une molécule de HLA, tandis que la partie comprend au moins 6 acides aminés consécutifs de l'antigène associé à une tumeur tel que défini sous (a),
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

2. Composition pharmaceutique comportant un anticorps qui se lie à la séquence d'acides aminés selon SEQ ID NO: 4 du listage de séquences.

3. Composition pharmaceutique selon la revendication 1, dans laquelle la cellule hôte sécrète l'antigène associé à une tumeur ou la partie de celui-ci, exprime sur la surface ou exprime une molécule de HLA qui se lie à l'antigène associé à une tumeur ou à la partie de celui-ci.

4. Composition pharmaceutique selon l'une des revendications 1-3 pour le traitement d'une maladie, qui se manifeste par l'expression ou l'expression anormale d'un antigène associé à une tumeur.

5. Procédé pour le diagnostic d'une maladie, qui se manifeste par l'expression ou l'expression anormale d'un antigène associé à une tumeur, comprenant
(i) la mise en évidence d'un acide nucléique qui code pour l'antigène associé à une tumeur ou une partie de celui-ci qui comprend au moins 6 acides aminés consécutifs de l'antigène associé à une tumeur tel que défini sous (a), et/ou
(ii) la mise en évidence de l'antigène associé à une tumeur ou d'une partie de celui-ci qui comprend au moins 6 acides aminés consécutifs de l'antigène associé à une tumeur tel que défini sous (a), et/ou
(iii) la mise en évidence d'un anticorps qui se lie à la séquence d'acides aminés selon SEQ ID NO: 4 du listage de séquences, et/ou
(iv) la mise en évidence de lymphocytes T cytotoxiques ou auxiliaires, qui sont spécifiques pour l'antigène associé à une tumeur ou une partie de celui-ci, tandis que la partie comprend au moins 6 acides aminés consécutifs de l'antigène associé à une tumeur tel que défini sous (a),
dans un échantillon biologique isolé à partir d'un patient,
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique selon (a) au moins 90% d'identité de séquences.

6. Procédé pour la détermination de la régression, de l'évolution ou de l'interruption d'une maladie, qui se manifeste par l'expression ou l'expression anormale d'un antigène associé à une tumeur, comprenant la surveillance d'un échantillon provenant d'un patient, qui présente la maladie ou est suspecté d'être atteint par la maladie, en ce qui concerne un ou plusieurs paramètres choisis dans le groupe consistant en:
(i) la quantité de l'acide nucléique qui code pour l'antigène associé à une tumeur, ou d'une partie de celui-ci,
(ii) la quantité de l'antigène associé à une tumeur ou d'une partie de celui-ci qui comprend au moins 6 acides aminés consécutifs de l'antigène associé à une tumeur, tel que défini sous (a),
(iii) la quantité des anticorps qui se lient à la séquence d'acides aminés selon SEQ ID NO: 4 du listage de séquences, et
(iv) la quantité de cellules T cytolytiques ou libérant des cytokines qui sont spécifiques pour un complexe entre l'antigène associé à une tumeur ou une partie de celui-ci et une molécule de CMH, tandis que la partie comprend au moins 6 acides aminés consécutifs de l'antigène associé à une tumeur tel que défini sous (a),
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

7. Anticorps qui se lie spécifiquement à la séquence d'acides aminés selon SEQ ID NO: 4 du listage de séquences.

8. Composition pharmaceutique selon la revendication 2 ou anticorps selon la revendication 7, dans laquelle/lequel l'anticorps est couplé avec un agent thérapeutique ou diagnostique.

9. Anticorps selon la revendication 8 pour utilisation dans un procédé pour le traitement, le diagnostic ou le suivi d'une maladie qui se manifeste par l'expression ou l'expression anormale d'un antigène associé à une tumeur, tandis que le procédé comprend l'administration de l'anticorps et que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

10. Anticorps selon la revendication 8 pour utilisation dans un procédé pour le traitement, le diagnostic ou le suivi d'une tumeur donnant des métastases qui se manifeste par l'expression ou l'expression anormale d'un antigène tumoral, tandis que le procédé comprend l'administration de l'anticorps et que l'antigène tumoral présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

11. Composition pharmaceutique selon la revendication 2 ou 8, composition pharmaceutique pour utilisation selon la revendication 4, anticorps selon la revendication 7 ou 8 ou anticorps pour utilisation selon la revendication 9 ou 10, dans laquelle/lequel l'anticorps est un anticorps monoclonal, chimérique ou humanisé ou un fragment d'un anticorps.

12. Cellules T cytolytiques ou libérant des cytokines pour utilisation dans un procédé pour le traitement d'un patient ayant une maladie qui se manifeste par l'expression ou l'expression anormale d'un antigène associé à une tumeur, comprenant:
(i) le prélèvement d'un échantillon avec des cellules immunoréactives à partir du patient,
(ii) la mise en contact de l'échantillon avec une cellule hôte, qui exprime l'antigène associé à une tumeur ou une partie de celui-ci, dans des conditions qui favorisent une production de cellules T cytolytiques ou libérant des cytokines contre l'antigène associé à une tumeur ou une partie de celui-ci, et
(iii) l'introduction des cellules T cytolytiques ou libérant des cytokines dans les patients en une quantité appropriée pour lyser les cellules qui expriment l'antigène associé à une tumeur ou une partie de celui-ci,
tandis que la partie comprend au moins 6 acides aminés consécutifs de l'antigène associé à une tumeur tel que défini sous (a),
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

13. Cellules T cytolytiques ou libérant des cytokines selon la revendication 12, dans lesquelles la cellule hôte exprime de manière recombinante ou endogène une molécule de HLA qui se lie à l'antigène associé à une tumeur ou à une partie de celui-ci.

14. Composition pharmaceutique selon la revendication 4, procédé selon la revendication 5 ou 6, anticorps selon l'une des revendications 9 à 11, ou cellules T cytolytiques ou libérant des cytokines selon la revendication 12 ou 13, dans laquelle/lequel la maladie est un cancer.

15. Composition pharmaceutique selon la revendication 4, procédé selon la revendication 5 ou 6, anticorps selon l'une des revendications 9 à 11, ou cellules T cytolytiques ou libérant des cytokines selon la revendication 12 ou 13, dans laquelle/lequel la maladie est un cancer du côlon, du rectum, du rein, du sein, de la prostate, de l'utérus, des ovaires, de l'endomètre, de l'oesophage, du sang, du foie, du pancréas, de la peau, du cerveau ou du poumon, un lymphome ou un neuroblastome, une tumeur du poumon, du sein, de la prostate, de l'ORL, de l'estomac, du côlon, un carcinome des cellules rénales, du col de l'utérus, du côlon ou mammalien, ou des métastases des types de cancers ou de tumeurs susdits.

16. Composition pharmaceutique selon l'une des revendications 1 à 4, 8, 9, 14 et 15, procédé selon l'une des revendications 5, 6, 14 et 15, anticorps selon l'une des revendications 7 à 11, 14 et 15, ou cellules T cytolytiques ou libérant des cytokines selon l'une des revendications 12 à 15, dans laquelle/lequel la protéine ou le polypeptide ou l'antigène associé à une tumeur comprend une séquence d'acides aminés qui est choisie dans le groupe consistant en les SEQ ID NOs: 4, 7 et 8 du listage de séquences.

17. Acide nucléique qui code pour une protéine ou un polypeptide, qui comprend une séquence d'acides aminés qui est choisie dans le groupe consistant en SEQ ID NOs: 4, 7 et 8 du listage de séquences.

18. Acide nucléique selon la revendication 17, qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique sous (a) au moins 90% d'identité de séquences.

19. Cellule hôte, qui comprend un acide nucléique selon la revendication 17 ou 18.

20. Protéine ou polypeptide, qui est codé(e) par un acide nucléique selon la revendication 17 ou 18.

21. Protéine ou polypeptide qui comprend une séquence d'acides aminés qui est choisie dans le groupe consistant en les SEQ ID NOs: 4, 7 et 8 du listage de séquences.

22. Kit pour la détection de l'expression ou l'expression anormale d'un antigène associé à une tumeur, comprenant des moyens pour la mise en évidence
(i) de l'acide nucléique qui code pour l'antigène associé à une tumeur ou une partie de celui-ci, qui comprend au moins 6 acides aminés consécutifs de l'antigène associé à une tumeur tel que défini sous (a),
(ii) de l'antigène associé à une tumeur ou d'une partie de celui-ci, qui comprend au moins 6 acides aminés consécutifs de l'antigène associé à une tumeur tel que défini sous (a),
(iii) d'anticorps qui se lient à la séquence d'acides aminés selon SEQ ID NO: 4 du listage de séquences, et/ou
(iv) de cellules T qui sont spécifiques pour un complexe entre l'antigène associé à une tumeur ou une partie de celui-ci, et une molécule de CMH, tandis que la partie comporte au moins 6 acides aminés consécutifs de l'antigène associé à une tumeur tel que défini dans (a),
tandis que l'antigène associé à une tumeur présente une séquence qui est codée par un acide nucléique qui est choisi dans le groupe consistant en:
(a) un acide nucléique qui comprend une séquence d'acide nucléique selon SEQ ID NO: 3 du listage de séquences,
et
(b) un acide nucléique qui présente par rapport à l'acide nucléique selon (a) au moins 90% d'identité de séquences.

23. Composition pharmaceutique qui comporte une interférence d'ARN (ARNi) qui empêche l'expression ou l'activité de l'antigène tumoral selon SEQ ID NO: 4 du listage de séquences.

24. Composition pharmaceutique selon la revendication 23, dans laquelle l'ARNi contient une courte structure en épingle à cheveu (shARN).


### SEQUENZPTOTOKOLL

<110> Ganymed Pharmaceuticals AG
<120> Identifizierung von tumorassoziierten zelloberflächen-Antigenen für die
   Diagnose und Therapie
<130> 342-10 EPT2
<150> DE 103 44 799.7
   <151> 2003-09-26
<160> 8
<170> Patentin Version 3.1
<210> 1
   <211> 2856
   <212> DNA
   <213> Homo sapiens
<400> 1
   atggccaaaa gaaatctcag cactgtgaca gagttcattc ttgtagtctt cacagatcac      60
   cctgaactgg cagttccact cttcctagtg tttctcagtt tctatcttgt cacttttctg     120
   gggaatgggg ggatgatcat tctaatccaa gtggatgccc aactccacac ccccgtgtac     180
   ttcttcctga gccaccttgc tttcctggat gcctgctgtg cctcagtaat cacccctcag     240
   attctggcca cactggccac agacaagaca gttatctcct atggctgccg tgctgtgcag     300
   ttctctttct tcaccatatg tgcaggcaca gagtgttacc tgctgtcagt gatggcctat     360
   gaccgctttg ttgccattag caatccactg cactgtaaca tgaccatgac tccaggtacc     420
   tgcagggtct ttttggccag tgccttcatc tgtggggtgt caggggccat tctgcatacc      480
   acgtgcacct tcaccctctc cttctgttgt gacaatcaga tcaacttctt cttctgtgac     540
   ctcccacccc tgctgaagct cgcctgcagc agcatgacac aaactgagat tgtcattctc     600
   ctttgtgcaa aatgcatgtt cctagccaat gtcatggtta tcctgatctg ctacatgctc 660
   attatcagag ccattttgag ggtgaagtcg gcaggaggcc tcctgatagc atctgctcat 720
   ttcgatgcat atgtatatga gacaggcatc aactacaaca cagtttatgg ctcaggaaag 780
   gcagtagggt ggtcctggag gagcctgcgg gaaaccaacc acatgagacc aggaaatact 840
   tcaaaacact cagcagccca gctgcatcaa tgcctcatcc agcaagttgg caggtggccc 900
   ttgcagagca tgcccttccc cgtttctgca gggccacctt ataagtcagt gcagcctctc 960
   cctggagacc cccggcctct cctgtgcatc accggattat ttctgacttt gaagatgatg 1020
   gggtgtgggc ccaggaggcc cagggacagg aagtctgact tcttcataaa cacagaccct 1080
   ggtgcagggt caccagaaga acagaggtgt ggatgggaag ggcatccttc ccactcctat 1140
   accctggggc tgtctctgcc agtcaacttc ggcctgaaat gtccatggtg gacactatct 1200
   ggacccccag ctacctgcca acgtccagac ctgcagacac cttctccacc aaaggagata 1260
   tgttcatccg ggctgcgacc ccttacacac agcgctggac cagacagaag tcaagttcca 1320
   gcagcctccg gagcagccac tatgctgaca aaggggctgc ccgacatcac tgtgggactg 1380
   cagatttatg actcctgcat ctcagggatc caggctctgg ggagcaccct ggccctgctg 1440
   tccaatcagc ttccacccac aaccaactat gcttgtggct cccagcaaca tctcctgggc 1500
   gtggttggag ggatgacctt cctggagtca gagcccatgt ctgagctgct ctccatctac 1560
   agagtccctc agggccaaag actcaccaaa aactttgaag taaaagaact tgtctgcaca 1620
   tatctggtag gacagcttcc ttatggcctg gtcagttatg acaacagcaa ctttgagtgg 1680
   ctggatcagc agctgcagaa gcagatcggg ggcgagggac ttcctgttgg cgctgcgccc 1740
   agccgtgtag ccaggcaaca gtctgatgag gaagctgtgg gaggagtgca gggatacagg 1800
   tggtctggat taggggcttc catccaaagt gccagagaag gggcttggca tcgcacaggg 1860
   ctggagaaca tgaccactgc ccacctgtct gccttcaaac ttcctgatct aactgccact 1920
   taccaagcct acctggcagc caaagccctg tgggttgcct atcagaactt gatgtcctgc 1980
   tctgagagag agggaccatt cctgggaggc acgtatgcca atgcatggga agccaggctt 2040
   tctcaggtta acttcaccac caaagcccaa gaagaggttt tcttcgccaa agatggggaa 2100
   gtgctgacaa cgtttgacat taaaaacatc tatgttctcc cagacctgtc aggacagaca 2160
   gccattgttg gacactttga cttcagagca ccttctggaa aagagcttct gttggatgac 2220
   agcgcaattg tctgggcaga aggaccctta aagattagag ctgagagaac cctaagaacc 2280
   aagaccacac agcacctctc acatcccaag ctccaggagt cccttcctct gtctgcaacg 2340
   aaaaacgtcc tgtggaaacc aggaagtcaa ccctatttga gaagtcaaaa tgctgctaca 2400
   aaagccttcc ctgacccaga agagaaatcg caatgtcacc agtttctctt tctcccttca 2460
   gatagtgttg catgtcagaa gtgctctgac aaccagtggc ccaatgtgca gaagggcgag 2520
   tgcatcccca aaacccttga cttcttgttc tatcacaagc cccttgacac agcgttggct 2580
   gtctgcacag ccctgctctt tctccttgcc ctggccatct taggcatctt ccatgttgtc 2640
   tgctcctgtg tctgggtgtc cttcatacct gcccacatgc atgcccacag caaagacacc 2700
   atggccatgg aggtctttgt catcttggca tcagcaggag gcctcatgtc ctccctcttc 2760
   ttttccaaat gctacatcat ccttctccat cctgaaaaga acacaaaaga ccaaatgttt 2820
   ggccggcatc atcgcaagtg ggaaaaactg aagtga 2856
<210> 2
   <211> 951
   <212> PRT
   <213> Homo sapiens
<400> 2
   Met Ala Lys Arg Asn Leu Ser Thr Val Thr Glu Phe Ile Leu Val Val
   1               5                   10                  15
   Phe Thr Asp His Pro Glu Leu Ala Val Pro Leu Phe Leu Val Phe Leu
               20                  25                  30
   Ser Phe Tyr Leu Val Thr Phe Leu Gly Asn Gly Gly Met Ile Ile Leu
           35                  40                  45
   Ile Gln Val Asp Ala Gln Leu His Thr Pro Val Tyr Phe Phe Leu Ser
       50                  55                  60
   His Leu Ala Phe Leu Asp Ala Cys Cys Ala Ser Val Ile Thr Pro Gln
   65                  70                  75                  80
   Ile Leu Ala Thr Leu Ala Thr Asp Lys Thr Val Ile Ser Tyr Gly Cys
                   85                  90                  95
   Arg Ala Val Gln Phe Ser Phe Phe Thr Ile Cys Ala Gly Thr Glu Cys
               100                 105                 110
   Tyr Leu Leu Ser Val Met Ala Tyr Asp Arg Phe Val Ala Ile Ser Asn
           115                 120                 125
   Pro Leu His Cys Asn Met Thr Met Thr Pro Gly Thr Cys Arg Val Phe
       130                 135                 140
   Leu Ala Ser Ala Phe Ile Cys Gly Val Ser Gly Ala Ile Leu His Thr
   145                 150                 155                 160
   Thr Cys Thr Phe Thr Leu Ser Phe Cys Cys Asp Asn Gln Ile Asn Phe
                   165                 170                 175
   Phe Phe Cys Asp Leu Pro Pro Leu Leu Lys Leu Ala Cys Ser Ser Met
               180                 185                 190
   Thr Gln Thr Glu Ile Val Ile Leu Leu Cys Ala Lys Cys Met Phe Leu
           195                 200                 205
   Ala Asn Val Met Val Ile Leu Ile Cys Tyr Met Leu Ile Ile Arg Ala
   210 215 220
   Ile Leu Arg Val Lys Ser Ala Gly Gly Leu Leu Ile Ala Ser Ala His
   225 230 235 240
   Phe Asp Ala Tyr Val Tyr Glu Thr Gly Ile Asn Tyr Asn Thr Val Tyr
   245 250 255
   Gly Ser Gly Lys Ala Val Gly Trp Ser Trp Arg Ser Leu Arg Glu Thr
   260 265 270
   Asn His Met Arg Pro Gly Asn Thr Ser Lys His Ser Ala Ala Gln Leu
   275 280 285
   His Gln Cys Leu Ile Gln Gln Val Gly Arg Trp Pro Leu Gln Ser Met
   290 295 300
   Pro Phe Pro Val Ser Ala Gly Pro Pro Tyr Lys Ser Val Gln Pro Leu
   305 310 315 320
   Pro Gly Asp Pro Arg Pro Leu Leu Cys Ile Thr Gly Leu Phe Leu Thr
   325 330 335
   Leu Lys Met Met Gly Cys Gly Pro Arg Arg Pro Arg Asp Arg Lys Ser
   340 345 350
   Asp Phe Phe Ile Asn Thr Asp Pro Gly Ala Gly Ser Pro Glu Glu Gln
   355 360 365
   Arg Cys Gly Trp Glu Gly His Pro Ser His Ser Tyr Thr Leu Gly Leu
   370 375 380
   Ser Leu Pro Val Asn Phe Gly Leu Lys Cys Pro Trp Trp Thr Leu Ser
   385 390 395 400
   Gly Pro Pro Ala Thr Cys Gln Arg Pro Asp Leu Gln Thr Pro Ser Pro
   405 410 415
   Pro Lys Glu Ile Cys Ser Ser Gly Leu Arg Pro Leu Thr His Ser Ala
   420 425 430
   Gly Pro Asp Arg Ser Gln Val Pro Ala Ala Ser Gly Ala Ala Thr Met
   435 440 445
   Leu Thr Lys Gly Leu Pro Asp Ile Thr Val Gly Leu Gln Ile Tyr Asp
   450 455 460
   Ser Cys Ile Ser Gly Ile Gln Ala Leu Gly Ser Thr Leu Ala Leu Leu
   465 470 475 480
   Ser Asn Gln Leu Pro Pro Thr Thr Asn Tyr Ala Cys Gly Ser Gln Gln
   485 490 495
   His Leu Leu Gly Val Val Gly Gly Met Thr Phe Leu Glu Ser Glu Pro
   500 505 510
   Met Ser Glu Leu Leu Ser Ile Tyr Arg Val Pro Gln Gly Gln Arg Leu
   515 520 525
   Thr Lys Asn Phe Glu Val Lys Glu Leu Val Cys Thr Tyr Leu Val Gly
   530 535 540
   Gln Leu Pro Tyr Gly Leu Val Ser Tyr Asp Asn Ser Asn Phe Glu Trp
   545 550 555 560
   Leu Asp Gln Gln Leu Gln Lys Gln Ile Gly Gly Glu Gly Leu Pro Val
   565 570 575
   Gly Ala Ala Pro Ser Arg Val Ala Arg Gln Gln Ser Asp Glu Glu Ala
   580 585 590
   Val Gly Gly Val Gln Gly Tyr Arg Trp Ser Gly Leu Gly Ala Ser Ile
   595 600 605
   Gln Ser Ala Arg Glu Gly Ala Trp His Arg Thr Gly Leu Glu Asn Met
   610 615 620
   Thr Thr Ala His Leu Ser Ala Phe Lys Leu Pro Asp Leu Thr Ala Thr
   625 630 635 640
   Tyr Gln Ala Tyr Leu Ala Ala Lys Ala Leu Trp Val Ala Tyr Gln Asn
   645 650 655
   Leu Met Ser Cys Ser Glu Arg Glu Gly Pro Phe Leu Gly Gly Thr Tyr
   660 665 670
   Ala Asn Ala Trp Glu Ala Arg Leu Ser Gln Val Asn Phe Thr Thr Lys
   675 680 685
   Ala Gln Glu Glu Val Phe Phe Ala Lys Asp Gly Glu Val Leu Thr Thr
   690 695 700
   Phe Asp Ile Lys Asn Ile Tyr Val Leu Pro Asp Leu Ser Gly Gln Thr
   705 710 715 720
   Ala Ile Val Gly His Phe Asp Phe Arg Ala Pro Ser Gly Lys Glu Leu
   725 730 735
   Leu Leu Asp Asp Ser Ala Ile Val Trp Ala Glu Gly Pro Leu Lys Ile
   740 745 750
   Arg Ala Glu Arg Thr Leu Arg Thr Lys Thr Thr Gln His Leu Ser His
   755 760 765
   Pro Lys Leu Gln Glu Ser Leu Pro Leu Ser Ala Thr Lys Asn Val Leu
   770 775 780
   Trp Lys Pro Gly Ser Gln Pro Tyr Leu Arg Ser Gln Asn Ala Ala Thr
   785 790 795 800
   Lys Ala Phe Pro Asp Pro Glu Glu Lys Ser Gln Cys His Gln Phe Leu
   805 810 815
   Phe Leu Pro Ser Asp Ser Val Ala Cys Gln Lys Cys Ser Asp Asn Gln
   820 825 830
   Trp Pro Asn Val Gln Lys Gly Glu Cys Ile Pro Lys Thr Leu Asp Phe
   835 840 845
   Leu Phe Tyr His Lys Pro Leu Asp Thr Ala Leu Ala Val Cys Thr Ala
   850 855 860
   Leu Leu Phe Leu Leu Ala Leu Ala Ile Leu Gly Ile Phe His Val Val
   865 870 875 880
   Cys Ser Cys Val Trp Val Ser Phe Ile Pro Ala His Met His Ala His
   885 890 895
   Ser Lys Asp Thr Met Ala Met Glu Val Phe Val Ile Leu Ala Ser Ala
   900 905 910
   Gly Gly Leu Met Ser Ser Leu Phe Phe Ser Lys Cys Tyr Ile Ile Leu
   915 920 925
   Leu His Pro Glu Lys Asn Thr Lys Asp Gln Met Phe Gly Arg His His
   930 935 940
   Arg Lys Trp Glu Lys Leu Lys
   945 950
<210> 3
   <211> 2856
   <212> DNA
   <213> Homo sapiens
<400> 3
   atggccaaaa gaaatctcag cactgtgaca gagttcattc ttgtagtctt cacagatcac     60
   cctgaactgg cagttccact cttcctagtg tttctcagtt tctatcttgt cacttttctg    120
   gggaatgggg ggatgatcat tctaatccaa gtggatgccc aactccacac ccccgtgtac    180
   ttcttcctga gccaccttgc tttcctggat gcctgctgtg cctcagtaat cacccctcag    240
   attctggcca cactggccac agacaagaca gttatctcct atggctgccg tgctgtgcag    300
   ttctctttct tcaccatatg tgcaggcaca gagtgttacc tgctgtcagt gatggcctat    360
   gaccgctttg ttgccattag caatccactg cactgtaaca tgaccatgac tccaggtacc    420
   tgcagggtct ttttggccag tgccttcatc tgtggggtgt caggggccat tctgcatacc     480
   acgtgcacct tcaccctctc cttctgttgt gacaatcaga tcaacttctt cttctgtgac    540
   ctcccacccc tgctgaagct cgcctgcagc agcatgacac aaactgagat tgtcattctc    600
   ctttgtgcaa aatgcatgtt cctagccaat gtcatggtta tcctgatctg ctacatgctc    660
   attatcagag ccattttgag ggtgaagtcg gcaggaggcc tcctgatagc atctgctcat    720
   ttcgatgcat atgtatatga gacaggcatc aactacaaca cagtttatgg ctcaggaaag    780
   gcagtagggt ggtcctggag gagcctgcgg gaaaccaacc acatgagacc aggaaatact    840
   tcaaaacact cagcagccca gctgcatcaa tgcctcatcc agcaagttgg caggtggccc    900
   ttgcagagca tgcccttccc cgtttctgca gggccacctt ataagtcagt gcagcctctc    960
   cctggagacc cccggcctct cctgtgcatc accggattat ttctgacttt gaagatgatg   1020
   gggtgtgggc ccaggaggcc cagggacagg aagtctgact tcttcataaa cacagaccct   1080
   ggtgcagggt caccagaaga acagaggtgt ggatgggaag ggcatccttc ccactcctat   1140
   accctggggc tgtctctgcc agtcaacttc ggcctgaaat gtccatggtg gacactatct   1200
   ggacccccag ctacctgcca acgtccagac ctgcagacac cttctccacc aaaggagata   1260
   tgttcatccg ggctgcgacc ccttacacac agcgctggac cagacagaag tcaagttcca   1320
   gcagcctccg gagcagccac tatgctgaca aaggggctgc ccgacatcac tgtgggactg   1380
   cagatttatg actcctgcat ctcagggatc caggctctgg ggagcaccct ggccctgctg   1440
   tccaatcagc ttccacccac aaccaactat gcttgtggct cccagcaaca tctcctgggc   1500
   gtggttggag ggatgacctt cctggagtca gagcccatgt ctgagctgct ctccatctac   1560
   agagtccctc agggccaaag actcaccaaa aactttgaag taaaagaact tgtctgcaca   1620
   tatctggtag gacagcttcc ttatggcctg gtcagttatg acaacagcaa ctttgagtgg   1680
   ctggatcagc agctgcagaa gcagatcggg ggcgagggac ttcctgttgg cgctgcgccc   1740
   agccgtgtag ccaggcaaca gtctgatgag gaagctgtgg gaggagtgca gggatacagg 1800
   tggtctggat taggggcttc catccaaagt gccagagaag gggcttggca tcgcacaggg 1860
   ctggagaaca tgaccactgc ccacctgtct gccttcaaac ttcctgatct aactgccact 1920
   taccaagcct acctggcagc caaagccctg tgggttgcct atcagaactt gatgtcctgc 1980
   tctgagagag agggaccatt cctgggaggc acgtatgcca atgcatggga agccaggctt 2040
   tctcaggtta acttcaccac caaagcccaa gaagaggttt tcttcgccaa agatggggaa 2100
   gtgctgacaa cgtttgacat taaaaacatc tatgttctcc cagacctgtc aggacagaca 2160
   gccattgttg gacactttga cttcagagca ccttctggaa aagagcttct gttggatgac 2220
   agcgcaattg tctgggcaga aggaccctta aagattagag ctgagagaac cctaagaacc 2280
   aagaccacac agcacctctc acatcccaag ctccaggagt cccttcctct gtctgcaacg 2340
   aaaaacgtcc tgtggaaacc aggaagtcaa ccctatttga gaagtcaaaa tgctgctaca 2400
   aaagccttcc ctgacccaga agagaaatcg caatgtcacc agtttctctt tctcccttca 2460
   gatagtgttg catgtcagaa gtgctctgac aaccagtggc ccaatgtgca gaagggcgag 2520
   tgcatcccca aaacccttga cttcttgttc tatcacaagc cccttgacac agcgttggct 2580
   gtctgcacag ccctgctctt tctccttgcc ctggccatct taggcatctt ccatgttgtc 2640
   tgctcctgtg tctgggtgtc cttcatacct gcccacatgc atgcccacag caaagacacc 2700
   atggccatgg aggtctttgt catcttggca tcagcaggag gcctcatgtc ctccctcttc 2760
   ttttccaaat gctacatcat ccttctccat cctgaaaaga acacaaaaga ccaaatgttt 2820
   ggccggcatc atcgcaagtg ggaaaaactg aagtga 2856
<210> 4
   <211> 951
   <212> PRT
   <213> Homo sapiens
<400> 4
   Met Ala Lys Arg Asn Leu Ser Thr Val Thr Glu Phe Ile Leu Val Val
   1               5                   10                  15
   Phe Thr Asp His Pro Glu Leu Ala Val Pro Leu Phe Leu Val Phe Leu
               20                  25                  30
   Ser Phe Tyr Leu Val Thr Phe Leu Gly Asn Gly Gly Met Ile Ile Leu
           35                  40                  45
   Ile Gln Val Asp Ala Gln Leu His Thr Pro Val Tyr Phe Phe Leu Ser
       50                  55                  60
   His Leu Ala Phe Leu Asp Ala Cys Cys Ala Ser Val Ile Thr Pro Gln
   65                  70                  75                  80
   Ile Leu Ala Thr Leu Ala Thr Asp Lys Thr Val Ile Ser Tyr Gly Cys
                   85                  90                  95
   Arg Ala Val Gln Phe Ser Phe Phe Thr Ile Cys Ala Gly Thr Glu Cys
   100 105 110
   Tyr Leu Leu Ser Val Met Ala Tyr Asp Arg Phe Val Ala Ile Ser Asn
   115 120 125
   Pro Leu His Cys Asn Met Thr Met Thr Pro Gly Thr Cys Arg Val Phe
   130 135 140
   Leu Ala Ser Ala Phe Ile Cys Gly Val Ser Gly Ala Ile Leu His Thr
   145 150 155 160
   Thr Cys Thr Phe Thr Leu Ser Phe Cys Cys Asp Asn Gln Ile Asn Phe
   165 170 175
   Phe Phe Cys Asp Leu Pro Pro Leu Leu Lys Leu Ala Cys Ser Ser Met
   180 185 190
   Thr Gln Thr Glu Ile Val Ile Leu Leu Cys Ala Lys Cys Met Phe Leu
   195 200 205
   Ala Asn Val Met Val Ile Leu Ile Cys Tyr Met Leu Ile Ile Arg Ala
   210 215 220
   Ile Leu Arg Val Lys Ser Ala Gly Gly Leu Leu Ile Ala Ser Ala His
   225 230 235 240
   Phe Asp Ala Tyr Val Tyr Glu Thr Gly Ile Asn Tyr Asn Thr Val Tyr
   245 250 255
   Gly Ser Gly Lys Ala Val Gly Trp Ser Trp Arg Ser Leu Arg Glu Thr
   260 265 270
   Asn His Met Arg Pro Gly Asn Thr Ser Lys His Ser Ala Ala Gln Leu
   275 280 285
   His Gln Cys Leu Ile Gln Gln Val Gly Arg Trp Pro Leu Gln Ser Met
   290 295 300
   Pro Phe Pro Val Ser Ala Gly Pro Pro Tyr Lys Ser Val Gln Pro Leu
   305 310 315 320
   Pro Gly Asp Pro Arg Pro Leu Leu Cys Ile Thr Gly Leu Phe Leu Thr
   325 330 335
   Leu Lys Met Met Gly Cys Gly Pro Arg Arg Pro Arg Asp Arg Lys Ser
   340 345 350
   Asp Phe Phe Ile Asn Thr Asp Pro Gly Ala Gly Ser Pro Glu Glu Gln
   355 360 365
   Arg Cys Gly Trp Glu Gly His Pro Ser His Ser Tyr Thr Leu Gly Leu
   370 375 380
   Ser Leu Pro Val Asn Phe Gly Leu Lys Cys Pro Trp Trp Thr Leu Ser
   385 390 395 400
   Gly Pro Pro Ala Thr Cys Gln Arg Pro Asp Leu Gln Thr Pro Ser Pro
   405 410 415
   Pro Lys Glu Ile Cys Ser Ser Gly Leu Arg Pro Leu Thr His Ser Ala
   420 425 430
   Gly Pro Asp Arg Ser Gln Val Pro Ala Ala Ser Gly Ala Ala Thr Met
   435 440 445
   Leu Thr Lys Gly Leu Pro Asp Ile Thr Val Gly Leu Gln Ile Tyr Asp
   450 455 460
   Ser Cys Ile Ser Gly Ile Gln Ala Leu Gly Ser Thr Leu Ala Leu Leu
   465 470 475 480
   Ser Asn Gln Leu Pro Pro Thr Thr Asn Tyr Ala Cys Gly Ser Gln Gln
   485 490 495
   His Leu Leu Gly Val Val Gly Gly Met Thr Phe Leu Glu Ser Glu Pro
   500 505 510
   Met Ser Glu Leu Leu Ser Ile Tyr Arg Val Pro Gln Gly Gln Arg Leu
   515 520 525
   Thr Lys Asn Phe Glu Val Lys Glu Leu Val Cys Thr Tyr Leu Val Gly
   530 535 540
   Gln Leu Pro Tyr Gly Leu Val Ser Tyr Asp Asn Ser Asn Phe Glu Trp
   545 550 555 560
   Leu Asp Gln Gln Leu Gln Lys Gln Ile Gly Gly Glu Gly Leu Pro Val
   565 570 575
   Gly Ala Ala Pro Ser Arg Val Ala Arg Gln Gln Ser Asp Glu Glu Ala
   580 585 590
   Val Gly Gly Val Gln Gly Tyr Arg Trp Ser Gly Leu Gly Ala Ser Ile
   595 600 605
   Gln Ser Ala Arg Glu Gly Ala Trp His Arg Thr Gly Leu Glu Asn Met
   610 615 620
   Thr Thr Ala His Leu Ser Ala Phe Lys Leu Pro Asp Leu Thr Ala Thr
   625 630 635 640
   Tyr Gln Ala Tyr Leu Ala Ala Lys Ala Leu Trp Val Ala Tyr Gln Asn
                   645                 650                 655
   Leu Met Ser Cys Ser Glu Arg Glu Gly Pro Phe Leu Gly Gly Thr Tyr
               660                 665                 670
   Ala Asn Ala Trp Glu Ala Arg Leu Ser Gln Val Asn Phe Thr Thr Lys
           675                 680                 685
   Ala Gln Glu Glu Val Phe Phe Ala Lys Asp Gly Glu Val Leu Thr Thr
       690                 695                 700
   Phe Asp Ile Lys Asn Ile Tyr Val Leu Pro Asp Leu Ser Gly Gln Thr
   705                 710                 715                 720
   Ala Ile Val Gly His Phe Asp Phe Arg Ala Pro Ser Gly Lys Glu Leu
                   725                 730                 735
   Leu Leu Asp Asp Ser Ala Ile Val Trp Ala Glu Gly Pro Leu Lys Ile
               740                 745                 750
   Arg Ala Glu Arg Thr Leu Arg Thr Lys Thr Thr Gln His Leu Ser His
           755                 760                 765
   Pro Lys Leu Gln Glu Ser Leu Pro Leu Ser Ala Thr Lys Asn Val Leu
       770                 775                 780
   Trp Lys Pro Gly Ser Gln Pro Tyr Leu Arg Ser Gln Asn Ala Ala Thr
   785                 790                 795                 800
   Lys Ala Phe Pro Asp Pro Glu Glu Lys Ser Gln Cys His Gln Phe Leu
                   805                 810                 815
   Phe Leu Pro Ser Asp Ser Val Ala Cys Gln Lys Cys Ser Asp Asn Gln
               820                 825                 830
   Trp Pro Asn Val Gln Lys Gly Glu Cys Ile Pro Lys Thr Leu Asp Phe
           835                 840                 845
   Leu Phe Tyr His Lys Pro Leu Asp Thr Ala Leu Ala Val Cys Thr Ala
       850                 855                 860
   Leu Leu Phe Leu Leu Ala Leu Ala Ile Leu Gly Ile Phe His Val Val
   865                 870                 875                 880
   Cys Ser Cys Val Trp Val Ser Phe Ile Pro Ala His Met His Ala His
                   885                 890                 895
   Ser Lys Asp Thr Met Ala Met Glu Val Phe Val Ile Leu Ala Ser Ala
               900                 905                 910
   Gly Gly Leu Met Ser Ser Leu Phe Phe Ser Lys Cys Tyr Ile Ile Leu
   915 920 925
   Leu His Pro Glu Lys Asn Thr Lys Asp Gln Met Phe Gly Arg His His
   930 935 940
   Arg Lys Trp Glu Lys Leu Lys
   945 950
<210> 5
   <211> 22
   <212> DNA
   <213> künstliche Sequenz
<220>
   <223> oligonukleotid
<400> 5
   aggtggtgtg acgctgctgc ta 22
<210> 6
   <213> 22
   <212> DNA
   <213> künstliche Sequenz
<220>
   <223> oligonukleotid
<400> 6
   tcttcttggg ctttggtggt ga 22
<210> 7
   <211> 13
   <212> PRT
   <213> Homo sapiens
<400> 7
   Met Ala Lys Gln Gly Glu Met Asn Thr Ser Thr Ser Cys
   1 5 10
<210> 8
   <211>
   <212> PRT
   <213> Homo sapiens
<400> 8